(19) **Europäisches Patentamt European Patent Office Office européen des brevets**

(11) **EP 4 313 014 B1**

(12) **EUROPEAN PATENT SPECIFICATION**

(45) Date of publication and mention of the grant of the patent:
**28.01.2026 Bulletin 2026/05**

(21) Application number: **22717656.7**

(22) Date of filing: **21.03.2022**

(51) International Patent Classification (IPC):
*A61K 31/197* ^(2006.01)   *A61K 31/155* ^(2006.01)
*A61K 9/20* ^(2006.01)   *A61K 9/00* ^(2006.01)
*A61K 31/554* ^(2006.01)   *A61K 9/24* ^(2006.01)

(52) Cooperative Patent Classification (CPC):
**A61K 9/209; A61K 9/0065; A61K 9/2086;**
**A61K 9/2095; A61K 31/155; A61K 31/197;**
**A61K 31/554;** A61K 9/2027; A61K 9/2054

(86) International application number:
**PCT/IB2022/052522**

(87) International publication number:
**WO 2022/200971 (29.09.2022 Gazette 2022/39)**

(54) **A METHOD FOR THE PRODUCTION OF GASTRORETENTIVE COMPACT MATRICES FOR THE CONTROLLED RELEASE OF ACTIVE SUBSTANCES AND COMPACT MATRICES THUS OBTAINED**

VERFAHREN ZUR HERSTELLUNG GASTRORETENTIVER KOMPAKTER MATRIZEN ZUR KONTROLLIERTEN FREISETZUNG VON WIRKSTOFFEN UND SO ERHALTENE KOMPAKTE MATRIZEN

PROCÉDÉ DE PRODUCTION DE MATRICES COMPACTES DE RÉTENTION GASTRIQUE POUR LA LIBÉRATION CONTRÔLÉE DE SUBSTANCES ACTIVES ET MATRICES COMPACTES AINSI OBTENUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2021 IT 202100006776**

(43) Date of publication of application:
**07.02.2024 Bulletin 2024/06**

(73) Proprietor: **Università degli Studi di Genova**
**16126 Genova (IT)**

(72) Inventors:
• **CAVIGLIOLI, Gabriele**
**16148 Genova (IT)**
• **BALDASSARI, Sara**
**16148 Genova (IT)**
• **ZUCCARI, Guendalina**
**16148 Genova (IT)**
• **AILUNO, Giorgia**
**16148 Genova (IT)**

(74) Representative: **Botti & Ferrari S.p.A.**
**Via Cappellini, 11**
**20124 Milano (IT)**

(56) References cited:
**EP-A1- 2 105 133**   **WO-A1-03/097018**
**WO-A1-2018/232413**   **CN-A- 103 585 098**

• **CAVIGLIOLI GABRIELE ET AL: "An innovative matrix controlling drug delivery produced by thermal treatment of DC tablets containing polycarbophil and ethylcellulose", INTERNATIONAL JOURNAL OF PHARMACEUTICS, ELSEVIER, NL, vol. 458, no. 1, 18 October 2013 (2013-10-18), pages 74 - 82, XP028774695, ISSN: 0378-5173, DOI: 10.1016/ J.IJPHARM.2013.10.014**

EP 4 313 014 B1

## Description

Field of application

The present invention relates to the chemical industry field in general, and in particular to the pharmaceutical industry field.

In particular, the invention refers to a method for the production of gastroretentive compact matrices, which can constitute tablets or devices that can be used for the release of active substances, characterized by a prolonged release, and to the compact matrices thus obtained. More in particular, the invention relates to a method for the preparation of gastroretentive compact matrices, which can constitute tablets or devices for the release of active substances, which provides for a step of compression of specific components and a step of heat treatment.

Prior art

It is known that pharmaceutical forms which can be orally administered are generally preferred for the purposes of a therapeutic treatment because of their many advantages, such as ease of administration, formulation flexibility, cost effectiveness, ease of storage and transport and high compliance of the patients. However, conventional drug-release systems cannot always solve problems deriving from a formulation intended for gastrointestinal administration, such as incomplete drug-release and the need for a frequent administration.

Thus, gastroretentive release systems ("Gastro Retentive Dosage Systems", GRDS) have been developed that have a gastric residence time prolonged up to several hours, an increased therapeutic drug effectiveness resulting from an improved drug absorption and suitability for a targeted release in the stomach and the first tract of the small intestine. Moreover, said systems may be developed so as to allow for a controlled drug release by a continuous release for a prolonged time at the desired rate.

GRDSs can be applied to drugs that are instable or poorly soluble at pHs found in the various intestinal tracts; said systems may be advantageous also when used with active substances susceptible to degradation by intestinal enzymes, which start to carry out their action before the drug can be absorbed through the intestinal wall, thereby leaving only a fraction of the administered dose available for the desired therapeutic action. Other active ingredients that can benefit from GRDSs are those having an absorption window in the first tract of the intestine. Some examples of said active ingredients are listed in the following table.

| Problem | Drugs | Examples of therapeutic applications |
|---|---|---|
| Local activity | Ranitidine, amoxicillin, levofloxacin, metronidazole, antiacids, misoprostol | Peptic ulcer disease and reflux esophagitis, eradication of *H. pylori* |
| Plasma level fluctuations | Ciprofloxacin, clarithromycin | Infections of the urinary, respiratory and gastrointestinal tract |
| Instability or poor solubility at intestinal pH | Verapamil, ofloxacin, amoxicillin | Infections of the urinary, respiratory and gastrointestinal tract |
| Limited intestinal absorption window | Pregabalin, cyclosporin, levodopa, baclofen, gabapentin, methotrexate | Fibromyalgia, diabetic peripheral neuropathy, postherpetic neuralgia |
| Poor absorption in the lower intestinal tract, or better absorption in the gastric section | Atenolol, furosemide, diazepam, verapamil, metronidazole, nitrofurantoin, ciprofloxacin, tetracycline | Hypertension, infections |
| Drugs degrading in the colon or which are not well absorbed - degradation by intestinal enzymes | Ranitidine, metformin hydrochloride | Peptic ulcer disease, diabetes |

(continued)

| Problem | Drugs | Examples of therapeutic applications |
|---|---|---|
| Drugs which negatively influence the normal colon microorganisms; adverse effects related to the situation of the drug reaching the distal portion of the gastrointestinal tract | Antibiotics | |

Classes of drugs for which GRDSs might be useful may include anxiolytics and antidepressants, antiasthma drugs, drugs for neuropathic pain, anti-inflammatory agents and myorelaxants.

Active ingredients for which GRDSs might be suitable include alprazolam, diazepam, lorazepam, chlordiazepoxide amodiaquine dihydrochloride dihydrate, ketotifen fumarate, theophylline, pregabalin, lornoxicam, tizanidine, chloroquine diphosphate, doxycycline, chloroguanide hydrochloride (proguanil HCl), 5-aminosalicylic acid, sulfasalazine, atorvastatin calcium salt, fluvastatin sodium salt, pravastatin sodium salt, scopolamine hydrobromide trihydrate, levodopa, benserazide hydrochloride, carbidopa, lansoprazole, omeprazole, clopidogrel hydrogen sulfate, gliclazide, propanolol, bupropion, guaifenesin, benzydamine, ketoconazole, 5-fluorouracil, ethinylestradiol, ciprofloxacin hydrochloride, indapamide, nicardipine hydrochloride, oxybutynin hydrochloride, pyridostigmine bromide, pseudoephedrine hydrochloride, tramadol hydrochloride, venlafaxine hydrochloride, linagliptin, canagliflozin, sitagliptin, tapentadol.

GRDSs may be also useful for the administration of food supplements, since they improve the absorption of nutrients in the stomach, in the duodenum and in the first tract of the intestine.

The physiology of the model of stomach motility is usually divided into 4 steps: step 1 (a rest period of 30-60 minutes with rare contractions); step 2 (20-40-minutes long, with intermittent contractions whose intensity and frequency gradually increase); step 3 ("housekeeper wave", 10-20-minutes long periods displaying intense and regular contractions allowing all the non-digested materials to be expelled from the stomach); and step 4 (a transition step of 0-5 minutes before the start of a new cycle).

Gastric emptying occurs both on a full stomach and on an empty stomach, but in the empty-stomach state the "housekeeper wave" induces emptying about every 120 minutes through the pylorus, whose diameter increases up to about 19 mm; on a full stomach, the "housekeeper waves" are not generated and the food stays for long in the stomach.

Various factors influence the performance of the gastroretentive dosage forms:

- size of the dosage unit: the dosage forms which become larger than the diameter of the pyloric sphincter ($12.8 \pm 7$ mm on average) may be temporarily retained in the stomach;

- geometry of the dosage form: expandable GRDSs that unfold in the form of a tetrahedron and of a ring are reported to have a better gastric retention at 24 h compared to other forms, such as continuous rod, planar disk, multilobed and cord.

- density: in low-density systems, the density of the dosage forms should be lower than that of the gastric fluid (1.004 g / $cm^3$) to float in the gastric environment; on the other hand, high-density systems (> 2.500 g / $cm^3$) may sink to the bottom of the stomach and prevent gastric emptying;

- calorie density of ingested food: in general, an increase of the calorie density significantly increases the gastric residence time (GRT);

- posture: in erect position, a floating system floats in the gastric fluid for a prolonged time period compared to the GRT of non-floating systems;

- sex and age: the female gender and elderly patients have a slower gastric emptying compared to male and young patients.

The current technologies employed for GRDSs are based on sinking, swelling, floating, mucoadhesion or magnetic properties.

Sinking systems, which have a higher density compared to gastric fluids, are retained on the bottom of the stomach, which is anatomically lower than the pyloric sphincter. Commonly used excipients include barium sulfate, zinc oxide, powder of iron and titanium dioxide. However, due to the need of including a large amount of excipients, these systems have the drawback of a low drug load.

Expandable GRDSs increase their volume while they are in the stomach, thereby becoming unable to go through the pylorus. The expansion of the system occurs with swelling or unfolding, which allow for modification of volume and shape.

Swellable systems use hydrophilic polymers (e.g. hydroxypropylmethylcellulose, polyethylene oxide and carbopol) which can absorb water from gastric fluids and increase their own volume, thereby releasing the drug by diffusion. For example, Chen et al. formulated gastroretentive tablets by direct compression of a combination of hydroxyethylcellulose and sodium carboxymethylcellulose, and assessed the dependence of the swelling index on the composition [Chen et al. 2015. Swelling/Floating Capability and Drug Release Characterizations of Gastroretentive Drug Delivery System Based on a Combination of Hydroxyethyl Cellulose and Sodium Carboxymethyl Cellulose. PLoS ONE 10(1):e0116914].

In systems that unfold, the polymer and the drug are combined in a folded/compressed structure, in general inside a gelatin capsule which dissolves in the stomach, thereby releasing the system, that takes its unfolded configuration. They are generally made of biodegradable polymers.

Low-density/floating systems are the most convenient and extensively studied gastroretentive dosage form and are classified as effervescent and non-effervescent.

Non-effervescent systems contain highly swellable cellulose derivatives or gelling polymers such as hydroxypropylmethylcellulose, hydroxypropylcellulose, hydroxyethylcellulose, sodium carboxymethylcellulose, polyethylene oxide, carrageenin, agar, cellulose acetate phthalate and alginic acid [Kim et al. 2018. Preparation and evaluation of non-effervescent gastroretentive tablets containing pregabalin for once-daily administration and dose proportional pharmacokinetics Int J Pharm 550(1-2):160-169]. They are formulated in mono- or bilayer tablets or in hollow microspheres. For example, Wei et al. [2014. Gastro-floating bilayer tablets for the sustained release of metformin and immediate release of pioglitazone: Preparation and in vitro/in vivo evaluation. Int J Pharm 476(1-2):223-231] developed a floating bilayer tablet comprising an immediate-release layer and a prolonged-release layer including a hydrophilic polymer to control the rate of drug release and give the tablet buoyancy. A gastroretentive dosage form was prepared by expanding a melt and modeling the expanded melt into a tablet: the expansion is performed by mechanically dispersing a gas in the melt, which then solidifies into a low-density system capable of floating on the fluid of the stomach [Fenyvesi et al. 2019. Prolonged-release, gastroretentive, moulded, solid dosage form and process for the preparation thereof. WO 2019/069108 Al].

The limitation of non-effervescent systems is that the release kinetics of the drug cannot be modified without modifying the floating properties of the dosage form.

Effervescent floating systems include a gas-producing agent such as a carbonate (sodium bicarbonate, calcium carbonate), sometimes associated to weak organic acids such as tartaric acid or citric acid, or volatile liquids, combined with hydrophilic polymers. When carbonate-based systems meet the gastric fluid, $CO_2$ is released, that remains trapped in the lattice of the hydrophilic polymer, thereby making the matrix floating [Baumgartner et al. 2000.Optimisation of floating matrix tablets and evaluation of their gastric residence time. Int J Pharm 195(1-2):125-135]. An increase of the amount of sodium bicarbonate in the matrix is connected to a reduced release rate, since $CO_2$ bubbles hinder the diffusion path of the drug [Jiménez-Martinez et al. 2008. Sustained delivery of captopril from floating matrix tablets. Int J Pharm 362(1-2):37-43; Kishan et al. 2013. Drug-excipient interaction during formulation development in vitro and in vivo evaluation of gastroretentive drug delivery system for nizatidine. Int J Pharm Sci Nanotech 6(4)]. Effervescent systems can be of the mono- or bilayer type, the latter including a conventionally formulated immediate release layer. For example, sodium bicarbonate was used to improve the GRT of an hydroxypropylmethylcellulose matrix by increasing the hydration volume and the surface of diffusion of the drug [Jiménez-Martinez et al. 2008. Sustained delivery of captopril from floating matrix tablets. Int J Pharm 362(1-2):37-43]. Fukuda et al. developed floating tablets prepared by hot extrusion using sodium bicarbonate and Eudragit polymers as matrix-forming agents [2006. Floating hot-melt extruded tablets for gastroretentive controlled drug release system. J Control Release 115(2):121-9].

An effervescent system can consist of multiple units, as in [Meka et al. 2009. Design and evaluation of polymeric coated minitablets as multiple unit gastroretentive floating drug delivery systems for furosemide. J Pharm Sci 98(6):2122-32]. A recent patent describes a push-pull osmotic system whose pull layer comprises a mixture of sodium bicarbonate and calcium carbonate, delimited by an elastic and permeable membrane [Shah et al. 2020. Self-Regulating Osmotic Gastroretentive Drug Delivery Systems. WO2020006278].

In the case of volatile liquids, ether or cyclopentane are loaded in an inflatable chamber and, volatilizing at body temperature, allow the swelling of the chamber in the stomach [Michaels et al. 1975. Integrated device for administering beneficial drug at programmed rate. US 3,901,232].

For low-density systems, the floating latency time (the time between the intake of the dosage form and the beginning of its flotation), the total floating time and the floating force (weight reduction over time) are normally assessed in simulated gastric fluid at 37°C. In general, low-density systems require a suitable volume of gastric fluid to float and function efficiently. Another limitation is the low mechanical strength of the swelled system. To address this issue, WO 2018/232413 Al (Sandhu et al. 2018. *Gastroretentive dosage forms for sustained drug delivery*) describes a gastroretentive tablet with a core of swellable matrix coated by a water insoluble, permeable, elastic polyacrylic membrane, that gives appropriate mechanical properties and release control properties.

*Raft-forming systems* are formulated with effervescent excipients and gelling polymers, such as sodium alginate, to obtain a prolonged release of the drug. When the system comes into contact with the gastric fluid, it swells and forms a viscous gel layer. The raft formed may remain intact in the stomach for several hours, thereby promoting the prolonged release of the drug, mainly antiacids [Hampson et al. 2010. Alginate-antiacid combinations: raft formation and gastric retention studies. Drug Dev Ind Pharm 36(5):614-23.] Unfortunately, these systems have the drawback of a low mechanical strength.

Superporous hydrogels, consisting of hydrophilic polymers, are interesting gastroretentive systems, thanks to their high mechanical strength and their elastic properties [Omidian et al. 2005. Advances in superporous hydrogels. J Control Release 102(1):3-12]. They can swell up to 100 times and reach enough mechanical strength to tolerate gastric contractions, thereby increasing GRT. El-Said et al. [2016. Baclofen novel gastroretentive extended release gellan gum superporous hydrogel hybrid system: in vitro and in vivo evaluation. Drug Deliv 23(1):101-12] tested the properties of formation of hydrogels of various polymers and gellan gum cross-linked with calcium chloride for preparing systems which have been retained in dog stomach for maximum 6 hours. The pH-sensitivity of superporous hydrogels is their main limitation.

Mucoadhesive systems comprise polymers such as carbopol, chitosan, sodium alginate, hydroxypropylmethylcellulose, polyethylene glycol and polyacrylic acid, and are designed to adhere to the surface of gastric epithelial cells and to prolong the GRT of the active ingredients. Various forms of gastrointestinal mucoadhesive dosage such as pearls, microspheres, film, capsules and tablets have been prepared [Patil et al. 2016. Recent advancements in mucoadhesive floating drug delivery systems: A mini-review. J Drug Deliv Sci Tech 31:65-71]. Before testing them in vivo, the rheological characterization of said systems is required.

Magnetic dosage forms include a small amount of magnetic material, sensitive to an extracorporeal magnet positioned above the stomach to control the position in the patient's gastrointestinal tract.

One of the main problems to solve in gastroretentive systems is the variation of performance, especially in the state of full stomach and empty stomach. This limitation may be overcome by associating the different mechanisms, since systems based on different principles of operation are less affected by physiological conditions.

For example, Sivaneswari et al. [2017. Novel expandable gastro retentive system by unfolding mechanism of levetiracetam using simple lattice design-Formulation optimization and in vitro evaluation. Bull Faculty Pharmacy, Cairo University 55(1):63-72] developed a new expandable GRDS based on a unfolding mechanism, wherein the drug is loaded on a polymeric patch of hydroxypropylmethylcellulose, carbopol and xanthan gum, designed to adhere to the gastric mucosa.

For in vitro assessment of GRDSs, some models of artificial stomach have been developed, for example by Kong e Singh [2008. A Model Stomach System to Investigate Disintegration Kinetics of Solid Foods during Gastric Digestion. J Food Sci 73(5):E202-10], Cascone et al. [2017. Mimicking the contractions of a human stomach and their effect on pharmaceuticals. J Drug Deliv Sci Tech 41:454-461], Lee et al. [2017. Application of an Artificial Stomach-Duodenum Reduced Gastric pH Dog Model for Formulation Principle Assessment and Mechanistic Performance Understanding. J Pharm Sci 106(8):1987-1997], or the TNO gastrointestinal model, or the dynamic gastric model of the English Institute of Food Research. These systems measure the forces which the food (and possibly the dosage forms) is subjected to in the stomach during digestion. GRDSs are assessed *in vivo* using various techniques of diagnostic imaging, including gamma scintigraphy, radiology, gastroscopy, ultrasonography and magnetic resonance imaging, both in animals (mainly rabbits and dogs), and in human volunteers.

Despite many attempts, in the art of modified-release dosage forms with rapid expansion the need to provide a prolonged release or to combine immediate and prolonged release of drugs requiring a gastric retention for about 8-16 h has still not been fully met. The object disclosed herein meets this need.

WO 2018/232413 describes floating gastroretentive tablets comprising hydroxypropylcellulose, carbopol and carbonates. Said tablets are coated at a temperature of 35-45°C.

CN 103 585 098 describes floating gastroretentive tablets comprising calcium phosphate, carbomer, Polycarbophil, hydroxypropylmethylcellulose, sodium bicarbonate. Said tablets are produced by wet granulation, followed by compression.

EP 2 105 133 describes gastroretentive tablets comprising Carbopol 971P, anhydrous calcium hydrogen phosphate and hydroxypropylmethylcellulose. The tablets are produced by direct compression.

WO 03/097018 describes gastroretentive tablets comprising dried glucose syrup, hydroxypropylcellulose, ammonium carbonate and calcium carbonate. The tablets, which do not contain a water-insoluble, water-swellable cross-linked polycarboxylic polymer, are heated up to 90°C.

Summary of the invention

A previous patent (EP 2 344 140 B1) described an innovative matrix, produced by heat treatment on direct compression (DC) tablets containing at least one alkylcellulose or one hydroxyalkylcellulose and a water-insoluble, water-swellable cross-linked polycarboxylic polymer. The compressed units thus obtained were able to control the release of the loaded drug, mainly at a constant rate in an aqueous solution at pH 4-8 even for 25 h.

Experimental evidence suggests that the heat treatment (80-250°C) acts on the tablet components generating a hydrophilic matrix that considerably swells in the pH range from 4 to 8, thereby controlling drug release. The hydrogel layer which has swollen around the swelling front controls the drug release by the so-called anomalous transport. In fact, the correlation between swelling and drug release profiles at pH 7.2 was perfect (Caviglioli et al. 2013 An innovative matrix controlling drug delivery produced by thermal treatment of DC tablets containing Polycarbophil and ethylcellulose. Int J Pharm 458(1):74-82).

The tablets according to the previous patent EP 2 344 140 B1 control better the drug release at pH > 4, since the polymer, which is pH-sensitive, forms at said values the gel layer capable of controlling the release. From this experimental evidence, it was thought to add one or more components that, even after the heat treatment, are able to adjust the pH of the acidic medium which diffuses in the pharmaceutical form to values higher than 4, thereby allowing gelling and therefore swelling of the matrix also in the acidic environment of the stomach, controlling, moreover, the rate and the time of drug release also at gastric level, i.e. in conditions in which the pH of the biological medium is lower than 4. In fact, the increase in size and the possible reduction of the density of the swollen matrix promote retention at gastric level of the pharmaceutical form. Moreover, the pharmaceutical form thus obtained has, due to its composition, also mucoadhesive properties that could promote its permanence in the stomach.

In the abovementioned paper, Caviglioli et al. describe a tablet produced according to the method described in EP 2 344 140 B1 and comprising 20% diltiazem hydrochloride, 20% Polycarbophil, 30% ethylcellulose and 30% Microcelac® and the modification of the composition of this tablet by substitution of Microcelac® with anhydrous dicalcium phosphate (calcium hydrogen phosphate), as an alternative diluent. However, in said paper there is no mention to the fact that the tablets therein described have gastroretentive characteristics.

In an aspect thereof, the present invention has the object of providing a compact matrix containing a cross-linked polycarboxylic polymer, non-erodible and bioadhesive, capable of swelling by water absorption also at gastric level, forming a non-erodible gel layer, and of being retained at gastric level, so as to be usable i.a. for the prolonged release of active substances.

Such an object is attained by means of a method for the preparation of a compact bioadhesive, gastroretentive matrix, which comprises the following steps:

- preparing a homogeneous mixture of powders comprising at least one alkylcellulose, a water-insoluble, water-swellable cross-linked polycarboxylic polymer and at least one pH regulator selected from the group consisting of carbonate and bicarbonate of an alkali metal or an alkaline earth metal, and hydrogen phosphate of an alkali metal;

- preparing compressed or compact units starting from said mixture of powders by direct compression or dry compaction;

- subjecting the compressed or compacted units thus obtained to heating at a temperature between 80°C and 250°C, preferably between 100°C and 160°C, for a time between 1 and 60 minutes.

In another aspect thereof, the invention has the object of providing a compressed unit, comprising the aforesaid compact, bioadhesive matrix, capable of swelling in water and of being retained at gastric level, for the release of active substances, characterized by a prolonged or controlled release. Such object is achieved by a method for the preparation of a compact matrix as described above, in which the aforesaid homogeneous mixture of powders also comprises at least one active substance.

The term "compressed unit" is intended to indicate not only the conventional tablets for pharmaceutical use, in particular those for oral administration capable of releasing active ingredients or substances which restore physiological conditions, but also other devices obtainable by powder compression, for example urethral suppositories, tablets and disks for vaginal, buccal, nasal, dental, otological, ophthalmic or even epidermal application, capable of releasing active ingredients or substances which restore physiological conditions. The application of such tablets and devices should not be intended as limited to the field of pharmaceutical products for human and veterinary use, where by active ingredient it is meant the medicinal substances according to the definition given respectively in EU Directive 2004/27/EC (art.1) and Regulation (EU) 2019/6, , but extended to other fields, such as that of medical devices according to the definition given in Regulation (EU) 2017/745, that of food according to the definition given in art.2 of the Regulation (EC) No. 178/2002, that of food supplements as defined by Directive 2002/46/EC, that of the dietary products (Regulation (EC) n. 1925/2006, Delegated Regulation (EU) 2016/128 and Regulation (EU) No. 609/2013) and of products for infants as defined by Regulation (EU) No. 609/2013, that of the plant protection products, according to the definition given in Regulation (EC) No. 1107/2009, that of manure or fertilizers according to the definition and classification of Regulation (EC) No. 2003/2003, that of disinfectants and insecticides and biocides in general according to the definition given in Regulation EU No. 528/2012, that of detergents according to the definition of Regulation (EC) No. 648/2004. Radiopharmaceuticals, radionuclides and molecules marked with radionuclides can also be carried and released by such matrix for diagnostic, therapeutic and general biocide purposes.

The aforesaid alkylcellulose can be selected, for example, from the group consisting of methylcellulose (CAS RN 9004-67-5) and ethylcellulose (CAS RN 9004-57-3).

It is also possible to use, in partial substitution of alkylcelluloses, the following substances, also in combination with each other: crospovidone, povidone (9003-39-8), Kollidon® VA64, cellulose acetate phthalate (CAS RN 9004-38-0), hypromellose phthalate (CAS RN 9050-31-1), polyvinyl alcohol (CAS RN 9002-89-5), polyvinyl acetate phthalate (CAS RN 34481-48-6), the various cyclodextrins (as described in the related monograph of the Handbook of Pharmaceutical Excipients ninth edition, Pharmaceutical Press), various types of methacrylate polymers also sold under the name of Eudragit (Röhm GmbH) such as for example those called E, L, S, RS, RL, PO, NE, RSPM, in the various types produced also by Eastman Chemical Company and BASF, glyceryl triacetate, triethyl citrate, tributyl citrate, acetyl triethyl citrate, acetyl tributyl citrate, dibutyl sebacate, diethyl phthalate, dibutyl phthalate, dioctyl phosphate, polyethylene glycol, polyethylene oxides (CAS RN 25322-68-3), calcium carboxymethylcellulose (CAS RN 9050-04-8), sodium carboxymethylcellulose (CAS RN 9004-32-4), inuline (CAS RN 9005-80-5), chitosan (CAS RN 9012-76-4) and its derivatives, guar gum (CAS RN 9000-30-0), xanthan gum (11138-66-2) and tragacanth gum (CAS RN 9000-65-1), carbomer (CAS RN 9003-01-04 and 96827-24-6), carrageenan (as described in the related monograph of the Handbook of Pharmaceutical Excipients ninth edition), alginic acid (CAS RN 9005-32-7), poloxamers (CAS RN 9003-11-6), aliphatic polyesters (as described in the related monograph of the Handbook of Pharmaceutical Excipients ninth edition), cellulose acetate butyrate, chitosan lactate, pectin, polyethylene-co-vinyl acetate, polyethylene, polyvinyl acetate-co-methacrylic acid, carnauba wax, butylated hydroxyanisole, butylated hydroxytoluene, ascorbyl palmitate, glyceryl palmitostearate, hydrogenated soybean and castor oil (Sterotek® K), glyceryl monostearate, d-$\alpha$-tocopherol (vitamin E), vitamin E succinate, vitamin E TPGS, methyl paraben, butyl stearate, stearyl alcohol, saccharose monopalmitate (Sucroester), glycerolesters and PEG esters (Gelucire 44/14), glyceryl palmitostearate (Precirol® ATO 5), mineral oil, castor oil and excipients known for forming effervescent mixtures or systems.

The aforesaid non-water-soluble, cross-linked polycarboxylic polymer that is swellable in water can be selected from carbopol and Polycarbophil and preferably consists of Polycarbophil (CAS registry number 9003-01-04).

The pH regulator is preferably selected from the group consisting of carbonate and bicarbonate of Li, Na, K, Ca and Mg and hydrogen phosphate of Li, Na and K.

The temperature at which the compressed units are heated is preferably in the range of 100-160°C and the heating time is conveniently in the range of 1-30 minutes, in particular 1-20 minutes. The heating speed for bringing the compressed units to the treatment temperature can vary from 1°C/minute to 50°C/minute.

The compression of the powders to be subjected to the subsequent heat treatment can be performed by working with pressures between 7000 and 50000 kg/cm$^2$. It is also possible to obtain compacts, with low tensile strength, also to be subjected to subsequent heat treatment, by working at pressures between 100 and 7000 kg/cm$^2$.

The form of the compressed units can be any regular three-dimensional geometric shape, and the weight can vary according to needs and use (human or veterinary) up to exceeding 100 grams for veterinary use.

These compressed units, in their composition, can be supplemented, if necessary, with all of the adjuvants which are typically employed in compression processes and known to those skilled in the art: glidants, lubricants, anti-adhesive agents, disintegrating agents and super-disintegrating agents, flavorings, coloring agents, sweeteners and adsorbents.

Such tablets can be coated with the classical methods for polymer film-coating and/or dry coating (Pharmaceutical Dosage Forms:Tablets Volumes 1,2,3, edited by H.A. Lieberman, L. Lachman, J.B. Schwartz, Dekker, second edition US 1989) in order to confer gastro-resistance, entero-solubility or environmental protection to the active ingredient.

The tablets according to the invention can be used as core or layer, containing or not containing the active ingredient, in order to obtain tablets known with the name of *inlay tablets, multilayer tablets* and *core-tablets* (Pharmaceutical Dosage Forms: Tablets Volume 1, edited by H.A. Lieberman, L. Lachman, J.B. Schwartz, Dekker, second edition US 1989). The different additional layers can have qualitative composition identical to that herein disclosed and/or a different content of active substances or an additional active substance, or they can be different matrices that have already been described or employed in this field.

In the case of *multilayer tablets,* the matrix according to the invention represents at least one layer of the multilayer tablet.In the case of *core-tablets,* the matrix described herein can represent the core and/or the crown layer, called outer layer or outlayer, with or without active ingredient or with different active ingredients for each layer.

In the case of *inlay tablets,* the matrix according to the invention can represent both the outlayer and the inlay and can contain or not contain the active ingredient or several ingredients. Even if the method according to the present invention is preferably a direct compression method, in certain situations, in order to overcome several drawbacks that the particle size of certain active ingredients could cause, the techniques known to those skilled in the art can still be used in the preparation of the tablets or the cores/inlay or of some layers. Such known techniques are called wet granulation (wet granulation, fluid-bed, granulation, spray-drying, spray-congealing) or dry granulation (dry granulation or roller compaction); or, possibly, the compression process can be applied to pellets subjected to a process, known as spheronization, which produces granules of spherical form and controlled size (Remington: The Science and Practice of Pharmacy, Pharmaceutical Press 21st edition 2011, chapter 45, page 903). The various granulation types obviously require a minimum addition of adjuvants or excipients necessary for these process types and known to those skilled in the art. In the case of active substances susceptible to oxidation, the heating can be conveniently carried out in inert atmosphere, for example in nitrogen atmosphere.

For volatile or sublimable active substances, or in any case when necessary, the heat treatment can be carried out in a natural or an inert atmosphere, pressurizing such atmosphere up to 0.5 MPa above the environmental pressure.

The cooling step after heating can occur naturally or in forced manner, for example controlling the cooling through the ventilation of dry air or inert gas ($N_2$, Ar, He) at room temperature, or of dry air or inert gas cooled to a temperature lower than room temperature.

After heating, a conditioning time to environmental conditions may be necessary before packaging which, depending on the chosen composition, may even last 24 hours. Generally, such waiting time does not affect the quality of the production, but it is in any case preferable to wait a standard time of 5 minutes.

A preferred powder composition for the use in the method according to the invention comprises active substance, ethylcellulose, Polycarbophil and a pH regulator selected from sodium carbonate, sodium bicarbonate and disodium hydrogen phosphate. Polycarbophil generally constitutes 15-75% by weight of the total weight of the powder mixture before compression and heat treatment, preferably 30-65%.

The pH regulator preferably constitutes 10-51% by weight of the total weight of the mixture before compression and heat treatment, conveniently 15-45%.

The pH regulator and Polycarbophil are generally present in a weight ratio ranging from 1:3 to 1:1.

Ethylcellulose preferably constitutes 0.1-80% by weight of the total weight of the mixture before compression and heat treatment, conveniently 10-20%.

Ethylcellulose and Polycarbophil are generally present in a weight ratio ranging from 1:10 to 4:1. The active substance is contained in the powder mixture in a quantity ranging from 0.001 ppm (parts per million) to 50% by weight of the total weight of the mixture before compression and heat treatment. In addition, within this percentage, the active substance can be mixed with suitable adjuvant substances for the solubilization process, such as for example surfactants or substances forming hydrotropic complexes or inclusion complexes; or with substances promoting the processes of gastro-intestinal absorption or in any case the transmucosal absorption of drugs, known as enhancers; or substances which physically or chemically stabilize the active ingredient.

The active substance can be of natural origin, synthetic or semi-synthetic with pharmacological action, employable for therapeutic, diagnostic or prophylactic use in humans or animals, or a substance of natural origin, synthetic or semi-synthetic, biologically, physically or chemically active and employable.

The only condition required for the use of an active substance in the method according to the present invention is that it has a sufficient thermo-stability at the heating conditions (temperature, time) involved by the method itself.

The compressed units according to the present invention may also release substances having a nutritional power, such as diet supplements for humans or animals, including both normal subjects and subjects suffering from chronic or acute pathological conditions.

The present invention also refers to a prolonged-release tablet, preferably of the multilayer or *core-tablet* type, having bioadhesion and gastroretention characteristics and comprising at least one active substance, at least one alkylcellulose, a water-insoluble, water-swellable cross-linked polycarboxylic polymer, preferably consisting of Polycarbophil, and at least one pH regulator selected from the group consisting of carbonate and bicarbonate of an alkali metal or an alkaline earth metal and hydrogen phosphate of an alkali metal, said tablet having a floating lag time of less than 60 seconds, preferably less than or equal to 20 seconds, more preferably less than or equal to 10 seconds, measured in 0.1 M HCl (1 L), pH 1.2, at 37°C, wherein said tablet is obtainable with the method according to appended claim 2 or according to any one of claims 3 to 9, when dependent on claim 2.

The aforesaid alkylcellulose is preferably selected from the group consisting of methylcellulose and ethylcellulose.

Preferably, said tablet has a total floating time of at least 4 hours, conveniently of at least 8 hours, measured in 0.1 M HCl (1 L), pH 1.2, at 37°C.

Preferably, the tablet according to the invention has a percentage swelling index (SI% - *swelling index %*) between 180% and 800%, conveniently between 350% and 700%, assessed at the end of a dissolution test performed in 0.1 M HCl (1 L), pH 1.2, at 37°C.

Preferably, the tablet has an erosion index (EI%) in the range of 0.1%-75%, conveniently 7%-40%, assessed at the end of a dissolution test performed in 0.1 M HCl (1 L), pH 1.2, at 37°C.

Preferably, Polycarbophil constitutes 15-75%, conveniently 30-65%, by weight, of the total weight of said tablet.

Preferably, the pH regulator constitutes 10-51%, conveniently 15-45%, by weight, of the total weight of said tablet.

Preferably, the pH regulator and Polycarbophil are present in a weight ratio ranging from 1:3 to 1:1.

Preferably, ethylcellulose constitutes 0.1-80%, conveniently 10-20%, by weight, of the total weight of said tablet.

Preferably, ethylcellulose and Polycarbophil are present in a weight ratio ranging from 1:10 to 4:1.

Preferably, the at least one active substance is contained in the tablet in an amount ranging from 0.001 ppm to 50% by weight of the total weight of the tablet.

Preferably, the at least one active substance is selected from the group consisting of baclofen, diltiazem, levodopa, metformin and pharmaceutically acceptable salts thereof.

Preferably, said tablet has a controlled release and a release kinetics of the active substance of substantially zero order in an aqueous solution at pH 1-8.

Preferably, in the tablet according to the invention the alkylcellulose is ethylcellulose, the water-insoluble, water-swellable cross-linked polycarboxylic polymer is Polycarbophil, and the at least one pH regulator is selected from the group consisting of carbonate and bicarbonate of Li, Na, K, Ca and Mg and hydrogen phosphate of Li, Na and K.

When the tablet according to the invention is of the *core-tablet* type, it has preferably a weight ratio between core and coating of between 1:1.5 and 1:3, conveniently between 1:1.8 and 1:2.2.

The variation of the ratios between the components indicated above, the conditions of compression of the powders or granulates and the heating conditions, temperature and heat treatment time, allow to control the rate at which the release of the active substance occurs and the floating of the form. Moreover, it was verified that a second heat treatment performed on tablets that have been already thermally treated does not further modify the morphological characteristics and the properties of floating, release control, swelling and erosion resistance.

Such a tablet is obtainable with the above-illustrated method.

Brief description of the figures

Fig. 1 is a thermogram showing the DSC and TGA profiles of sodium carbonate.

Fig. 2 is a thermogram showing the DSC and TGA profiles of sodium bicarbonate.

Fig. 3 shows the release profiles of diltiazem hydrochloride (DTZ) in 0.1 N HCl, at pH 1.2, of tablets obtained with the method according to the present invention (example 1) compared with tablets of identical composition but not subjected to heat treatment and thus not according to the invention.

Fig. 4 shows average swelling profiles in acidic environment of tablets obtained with the method according to the present invention (example 1) at different compression forces.

Fig. 5 shows the release profiles of DTZ in 0.1 N HCl, pH 1.2, of tablets obtained with the method according to the present invention (example 2), containing $Na_2CO_3$, at different heat treatment temperatures compared with tablets of identical composition but not subjected to heat treatment and thus not according to the invention.

Fig. 6 shows the release profiles of DTZ in 0.1 N HCl, pH 1.2, of tablets obtained with the method according to the present invention (example 3), containing $Na_2CO_3$, at different heat treatment temperatures compared with tablets of identical composition but not subjected to heat treatment and thus not according to the invention.

Fig. 7 shows the release profiles of DTZ in 0.1 N HCl, pH 1.2, of tablets obtained with the method according to the present invention (example 4), containing $NaHCO_3$, at different heat treatment temperatures compared with tablets of identical composition but not subjected to heat treatment and thus not according to the invention.

Fig. 8 shows the swelling profiles of tablets obtained with the method according to the present invention (example 4), containing $Na_2CO_3$ and $NaHCO_3$, respectively.

Fig. 9 shows the release profile of DTZ of tablets according to the present invention (example 5), containing $NaHCO_3$ compared with tablets of identical composition but not subjected to heat treatment and thus not according to the invention, with pH change after 2 hours.

Fig. 10 comprises two photographs, each illustrating three tablets according to the invention (example 5) recovered at the end of the dissolution test with pH change of Fig. 9.

Fig.11 shows the release profile of DTZ from tablets according to the present invention (example 6) containing $Na_2HPO_4$ compared to that of tablets according to the invention containing $Na_2CO_3$ and $NaHCO_3$, respectively.

Fig. 12 is a photograph showing three tablets according to the invention (example 6) recovered at the end of the dissolution test with pH change.

Fig. 13 shows the release profile of baclofen in 0.1 N HCl, pH 1.2, of tablets according to the invention (example 7) containing $Na_2CO_3$ compared with tablets of identical composition but not subjected to heat treatment and thus not according to the invention.

Fig. 14 shows the release profile of baclofen in 0.1 N HCl, pH 1.2, from tablets according to the invention (example 7) containing $NaHCO_3$ compared with tablets of identical composition but not subjected to heat treatment and thus not according to the invention.

Fig. 15 shows the release profile of DTZ in 0.1 N HCl, pH 1.2, from bilayer and tri-layer tablets according to the invention (example 8).

Fig. 16 shows the release profile of metformin hydrochloride in 0.1 N HCl, pH 1.2, from tablets coated according to the invention (example 9) compared with tablets of identical composition but not subjected to heat treatment and thus not according to the invention.

Fig. 17 shows the release profile of metformin hydrochloride in 0.1 N HCl, pH 1.2, from two types of tablets coated according to the invention (ME8 and ME9 - examples 9 and 10) and from their co-administration.

Fig. 18 shows the release profile of metformin hydrochloride from the same coated tablets (and their co-administration) of Fig. 17 with pH change to 6.8 after 4 hours.

Fig. 19 shows the release profile of metformin hydrochloride in 0.1 N HCl, pH 1.2, from the co-administration of different types of tablets coated according to the invention (ME8 + ME9, ME10 or ME11 - example 10).

Fig. 20 shows the release profile of baclofen in 0.1 N HCl, pH 1.2, from tablets of 13-mm diameter coated according to the invention (example 11) compared with tablets of identical composition but not subjected to heat treatment and thus not according to the invention.

Fig. 21 shows the swelling profile in 0.1 N HCl, pH 1.2, of the tablets of Fig. 20.

Fig. 22 shows the profile of thickness variation in 0.1 N HCl, pH 1.2, of the tablets of Fig. 20.

Fig. 23 shows the profile of diameter variation in 0.1 N HCl, pH 1.2, of the tablets of Fig. 20.

Fig. 24 shows the release profile of baclofen in 0.1 N HCl, pH 1.2, from tablets having a 7-mm core and total diameter of 10 mm which are coated according to the invention (example 11), both thermally treated (according to the invention) and not subjected to heat treatment (not according to the invention), compared with tablets of identical composition but with 9-mm core and total diameter of 13 mm.

Fig. 25 shows the release profile of baclofen in 0.1 N HCl, pH 1.2, from tablets coated according to the invention (example 11) subjected to heat treatment of different duration.

Fig. 26 shows the release profile of baclofen from tablets coated according to the invention (example 11) with pH change to 6.8 after 4 hours.

Fig. 27 is a photograph showing two tablets according to the invention (example 11) recovered at the end of the dissolution test with pH change.

Fig. 28 is a photograph showing a tablet according to the invention after 4 hours of treatment in the DGM apparatus in the state simulating the "on an empty stomach" condition.

Fig. 29 is a photograph showing a tablet according to the invention after 6 hours of treatment in the DGM apparatus in the state simulating the "on a full stomach" condition.

Fig. 30 shows the release profile of DTZ in 0.1 N HCl, pH 1.2, from tablets coated according to the invention (example 13) compared with tablets of identical composition but not subjected to heat treatment and thus not according to the invention.

Fig. 31 shows the release profile of levodopa in 0.1 N HCl, pH 1.2, from coated tablets according to the invention (example 14) compared with tablets of identical composition but not subjected to heat treatment and thus not according to the invention.

Fig. 32 shows the profile of diameter variation in 0.1 N HCl, pH 1.2, of the baclofen tablets of 10-mm diameter of example 15.

Fig. 33 shows the swelling profile in 0.1 N HCl, pH 1.2, of the baclofen tablets of example 15.

Fig. 34 shows the profile of diameter variation in 0.1 N HCl, pH 1.2, of the tablets lacking active substance of example 16.

Fig. 35 shows the swelling profile in 0.1 N HCl, pH 1.2, of the tablets lacking active substance of example 16.

Detailed Description of the Invention

Based on a pre-formulation study, some biocompatible pH regulators suitable for pharmaceutical use were selected that, after heat treatment (which is fundamental for obtaining the matrix), maintain the ability to adjust the pH to values higher than 4 when the gastric fluid permeates into the matrix of the tablet. In fact, the matrix efficiently swells when the aqueous microenvironment that soaks it is at a pH higher than 4.

These include carbonates, bicarbonates and phosphates of various metals, such as for example $NaHCO_3$ and $Na_2CO_3$.

For example, Tab. 1 shows the abilities of some of the abovementioned salts to give a pH higher than 4 if dissolved in water after heat treatment at 150°C for 15 min.

*Tabella 1*

| | Solubility in 0.1 N HCl before heat treatment | pH in water before heat treatment | Solubility in 0.1 N HCl after heat treatment | pH in water after heat treatment |
|---|---|---|---|---|
| $Na_2CO_3$ | yes | 10 | yes | 10 |
| $NaHCO_3$ | yes | 7 | yes | 10 |
| $Na_2HPO_4\ 2H_2O$ | yes | 6 | yes | 6 |
| $Na_3PO_4\ 12H_2O$ | yes | 10 | yes | 10 |
| $MgCO_3$ | no | 6 | yes | 7 |

In the case of carbonates and bicarbonates, these have also the function of reducing the density of the pharmaceutical form due to $CO_2$ loss. This happens because of the reaction with HCl contained in the gastric fluid in the case of carbonate or, in the case of bicarbonate, because of the temperatures reached during the heat treatment to obtain the matrix of the previous patent.

In fact, $NaHCO_3$ starts to decompose at temperatures higher than 100°C (Keener et al. 1985. Thermal decomposition of sodium bicarbonate. Chem. Eng. Commun. 33:93-105),

$$2NaHCO_3\ (s) \rightarrow Na_2CO_3\ (s) + H_2O\ (g) + CO_2\ (g)$$

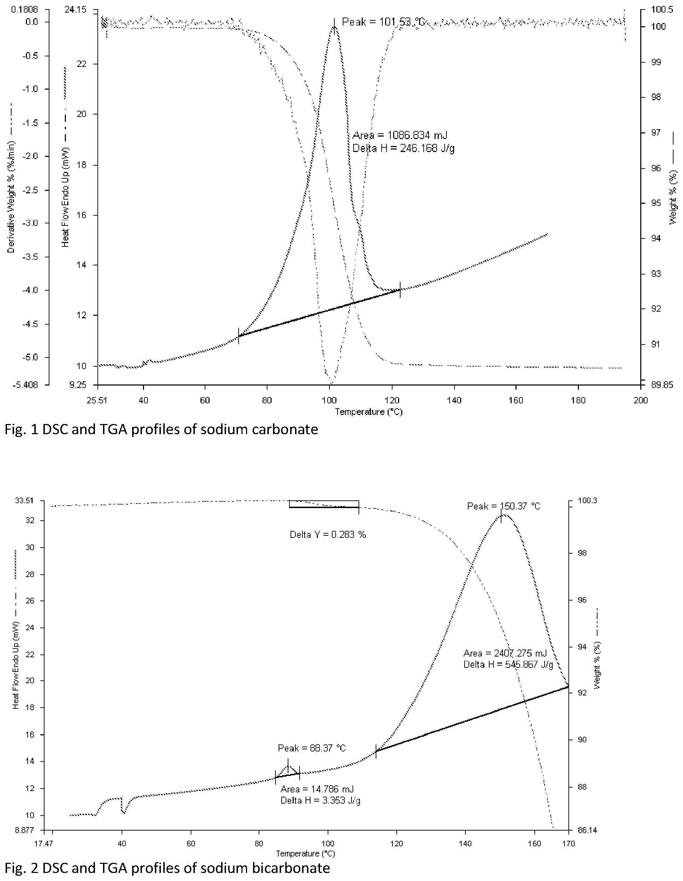

Instead, sodium carbonate is stable at the heat treatment conditions provided by this process; in fact, it decomposes only after fusion, which occurs at 851°C. In fact, as shown by the DSC and TGA analysis (Fig. 1), it is stable in the heat treatment conditions of this process. In the two traces of Fig. 1, only one thermal event associable to water loss, as determined with Karl Fisher method, is detected in the non-anhydrous samples of sodium carbonate.

As also evident in Fig. 2, practically, the heat treatment required to form the matrix allow the simultaneous decomposition of bicarbonate into $CO_2$ and $Na_2CO_3$, which causes a diminution of matrix density. The remaining $Na_2CO_3$, in contact with the gastric fluids, salifies the remaining carboxylic groups of the polyacrylic polymer thereby promoting matrix swelling, and thus both gastroretention and control of drug release, in addition to neutralizing the acidity of the gastric fluid that diffuses inside the matrix.

The matrix according to the patent application is capable of swelling, and therefore of controlling the release, in acidic (gastric) environment since the composition contains substances capable of adjusting the pH of the acidic solution, which diffuses toward the inside, to a pH capable of ionizing the carboxylic groups of the cross-linked polycarboxylic acid.

Initially, sodium carbonate and bicarbonate were used, to take advantage also of the reduction of density caused by the reaction with HCl and, in the case of bicarbonate, also of the increase in porosity of the matrix caused by a partial/total decomposition of the same during the heat treatment.

In fact, these substances, in addition to activating drug release even at the acidic pH that is typical of the gastric environment, causing the matrix to swell, reduce its weight, thereby promoting floating of the form in the gastric content, and the concurrent size increase that impedes it to go through the pyloric sphincter.

With said pH regulators, the heat treatment can control and/or reduce the rate of drug release even under conditions of gastric pH. Said effect is fundamental to increase the bioavailability of drugs with a limited intestinal absorption window, for example due to the presence of saturable absorption carriers in a certain tract of the intestine, or to disadvantageous pH conditions, or due to the presence of enzymatic activities.

Moreover, the different heat treatment, besides modulating the rate of drug release, modifies the erodibility of the form swelling in the gastric environment, that, due to its better resistance to peristalsis mechanical waves, has an increased gastric residence time.

Forms containing diltiazem hydrochloride subjected to dissolution test with pH change at 4 hours (modified USP methodology) have shown to be able to release in acidic environment about 40-50 % of the drug contained therein and, subsequently, also to control the release of the remaining DTZ at pH 6.8.

Some forms containing baclofen HCl were also designed. This derivative of the γ-aminobutyric acid, spasmolytic agent with central activity, has carriers for duodenal absorption.

The therapeutic importance of said molecule is evident from the many published papers and patents: it is useful in spasticities of subjects with spine injuries, in infantile cerebral palsies and it is recently being used also in alcoholism. It has several problems if administered orally in a conventional form, for this reason many authors consider suitable a GRT form.

Very important results were obtained with these and other drugs: slowdown of the release and/or control of the release rate; adjustability of the release depending on different heat treatments; size increase, floating capability, poor erodibility of the swelled matrix.

EXAMPLE 1

A comparison was made between two compacts containing sodium carbonate as a pH regulator, cylinder-shaped with 13-mm diameter and 300-mg average weight, whose composition is described in Tab. 2, obtained with two different compression forces and subjected to the same heat treatment of 150°C× 15 min.

*Table 2*

| Composition and characteristics of the compacts of batches X9A305 and X9A306 | | | | | | | | |
|---|---|---|---|---|---|---|---|---|
| Batch | Theoretical compression force (kN) | Mixture code | Tablet composition (%) | | | | Measured average compression force (kN±SD) | Average weight (mg ±SD) |
| X9A305 | 15 | 061212A | DTZ | Na$_2$CO$_3$ | POL | EC4 | 14.3±1.5 | 299.4±2.2 |
| | | | 20 | 30 | 45 | 5 | | |
| X9A306 | 25 | 061212A | DTZ | Na$_2$CO$_3$ | POL | EC4 | 25.4±2.7 | 301.3±3.1 |
| | | | 20 | 30 | 45 | 5 | | |
| *SD =standard deviation; DTZ = diltiazem hydrochloride; POL = Polycarbophil; EC4 = ethylcellulose Ethocel Standard 4 Premium Colorcon | | | | | | | | |

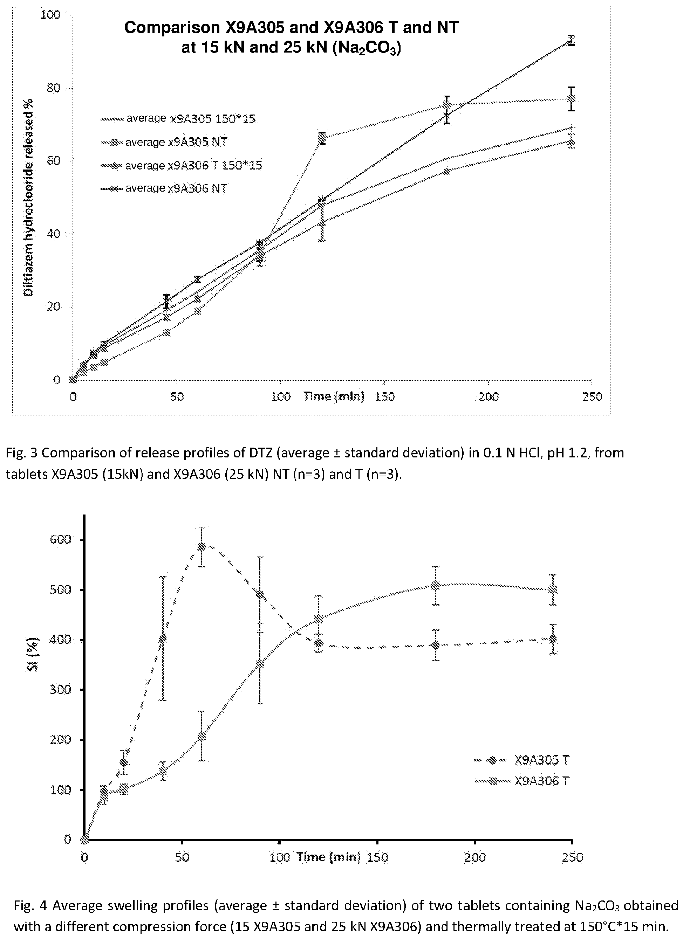

Fig. 3 shows the comparison of the release profiles of DTZ in 0.1 N HCl, pH 1.2, from disks subjected to a compression force of 15 kN (X9A305) and 25 kN (X9A306) thermally treated (T) and not thermally treated (NT).

Heat treatment at 150°C×15 min gives swelling ability and erosion resistance. NTs tend to disintegrate before swelling and for this reason do not control drug release.

From the point of view of the swelling in acidic environment, as shown in Fig. 4, the compacts obtained with 15 kN and thermally treated swell faster than the compacts obtained with 25 kN. Tablets containing $Na_2CO_3$ obtained with lower compression force swell earlier, increasing their weight by about 6 times after 50 min; those obtained with 25 kN reach the maximum after 120 min.

Thermally treated (T) tablets float after 10 min (X9A306) and 20 min (X9A305). X9A306 NTs start floating after 90 min. NT tablets obtained with low compression float immediately but erode almost double the amount of the tablets obtained with 25 kN and treated with 150°C×15 min (Tab. 3).

*Table 3*

| Erosion index (EI) of X9A305 and X9A306 compacts that were subjected to different heat treatments, recovered at the end of the dissolution test of Fig. 3 | | | | |
|---|---|---|---|---|
| | **X9A305 NT** | **X9A305 150°Cx15 min** | **X9A306 NT** | **X9A306 150°Cx15 min** |
| **EI% (average±SD)** | 84.0±2.3 | 47.0±9.9 | 53.2±6.5 | 40.4±4.3 |

All the NT and T compacts give effervescence in acidic environment since sodium carbonate does not decompose during heat treatment.

EXAMPLE 2

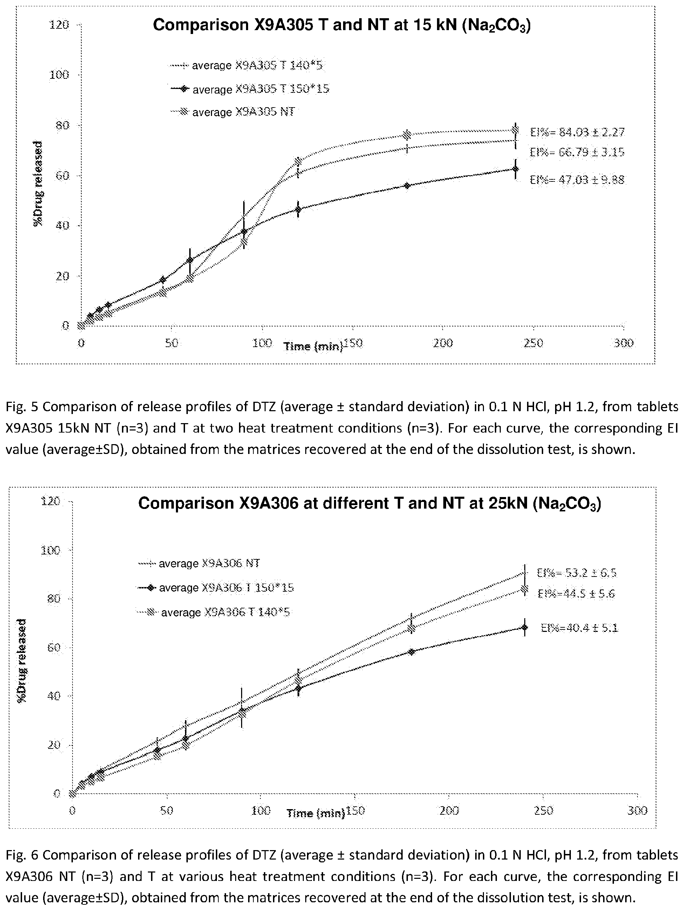

This example shows, with reference to Fig. 5, a comparison of the release profiles of DTZ from cylindrical tablets subjected to different heat treatments, containing sodium carbonate as a pH regulator, with 13-mm diameter and 300-mg average weight, whose composition is described in Tab. 2, obtained with a compression force of about 15 kN.

The floating behavior in 0.1 N HCl, pH 1.2, of the aforesaid tablets is deduced from Table 4.

*Table 4*

| | **Times (min)** | | | |
|---|---|---|---|---|
| | **0** | **10** | **20** | **90** |
| **X9A305 NT** | F1 | F1 | F1 | F1 |
| **X9A305 T 140°Cx5 min** | F1 | F1 | F1 | F1 |
| **X9A305 T 150°Cx15 min** | NF1 | NF1 | F1 | F1 |
| FI = floating; NF1 = not floating | | | | |

Table 5 shows the erosion index (EI) of the aforesaid tablets recovered at the end of the dissolution test of Fig. 5.

*Table 5*

| | **X9A305 NT** | **X9A305 140°Cx5 min** | **X9A305 150°Cx15 min** |
|---|---|---|---|
| **EI% (average ±SD)** | 84.0±2.3 | 66.8±3.2 | 47.0±9.9 |

As is deduced from the tables above, the heat treatment of X9A305 tablets obtained with 15 kN and containing $Na_2CO_3$ progressively reduces the erodibility of the matrix (Tab. 5) and causes the release of the drug to get close to a zero-order kinetics (Fig. 5). The compacts treated at 140°C for 5 min immediately float in the dissolution medium, whereas those treated at 150°C for 15 min float within 20 min.

EXAMPLE 3

This example presents, with reference to Figure 6, a comparison of the release profiles of DTZ from cylindrical tablets subjected to different heat treatments, containing sodium carbonate as a pH regulator, having cylindrical shape, with 13-mm diameter and 300-mg average weight, whose composition is described in the following Table 6, obtained with a compression force of about 25 kN.

Table 6

| Composition and characteristics of the X9A306 compacts | | | | | | | | |
|---|---|---|---|---|---|---|---|---|
| Batch | Theoretical compression force (kN) | Mixture code | Tablet composition (% w/w) | | | | Measured average compression force (kN$\pm$SD) | Average weight (mg $\pm$SD) |
| X9A306 | 25 | 061212A | DTZ | Na$_2$CO$_3$ | POL | EC4 | 25.4$\pm$2.7 | 301.3+3.1 |
| | | | 20 | 30 | 45 | 5 | | |
| *SD =standard deviation; DTZ = diltiazem hydrochloride; POL = Polycarbophil; EC4 = ethylcellulose Ethocel Standard 4 Premium Colorcon | | | | | | | | |

Fig. 6 shows the comparison of the release profiles of DTZ in 0.1 N HCl, pH 1.2, from cylindrical tablets subjected to a compression force of 25 kN (X9A306) subjected to different conditions of heat treatment or not subjected to heat treatment (NT).

The floating behavior in 0.1 N HCl, pH 1.2, of the aforesaid tablets during the dissolution test of Fig. 6 is deduced from Table 7.

Table 7

| | Times (min) | | | |
|---|---|---|---|---|
| | 0 | 10 | 20 | 90 |
| X9A306 NT | NF1 | NF1 | NF1 | F1 |
| X9A306 T 140°Cx5 min | NF1 | NF1 | NF1 | F1 |
| X9A306 T 150°Cx15 min | NF1 | F1 | F1 | F1 |
| F1 = floating; NFI = not floating | | | | |

It is noted that compacts treated at 150°C for 15 min float within 10 minutes, whereas NT tablets or tablets treated at 140°C $\times$ 5 min do not float until after 90 min from the start of the dissolution test.

Table 8 shows the erosion index (EI) of the aforesaid tablets recovered at the end of the dissolution test of Fig. 6.

Table 8

| | X9A306 NT | X9A306 140°C $\times$ 5 min | X9A306 150°C $\times$ 15 min |
|---|---|---|---|
| EI% (average$\pm$SD) | 53.2 $\pm$ 6.5 | 44.5 $\pm$ 5.6 | 40.4 $\pm$ 5.1 |

As is deduced from Table 8, the heat treatment of X9A306 tablets containing Na$_2$CO$_3$ progressively reduces the erodibility of the matrix and improves the control of drug release, slowing it down.

As highlighted in Examples 2 and 3 for compacts containing sodium carbonate as pH regulator obtained at both low compression force and higher compression force, the matrices show different responses depending on the different heat treatments, in particular they are adjustable, with the heat treatment and the compression force, the release profile of the drug depending on the different swelling capability, buoyancy and erosion resistance.

EXAMPLE 4

Different heat treatments on tablets containing sodium bicarbonate as a buffering agent, cylinder-shaped, with 13-mm diameter and 300-mg average weight, whose composition is described in Table 9, obtained with a compression force of about 25 kN, were compared.

*Table 9*

| Batch | Theoretical compression force (kN) | Mixture code | Tablet composition (% w/w) | | | | Measured average compression force (kN±SD) | Average weight (mg ±SD) |
|---|---|---|---|---|---|---|---|---|
| | | | Composition and characteristics of the X9A307 compacts | | | | | |
| X9A307 | 25 | 061212B | DTZ | NaHCO$_3$ | POL | EC4 | 26.0±3.7 | 301.6±1.0 |
| | | | 20 | 30 | 45 | 5 | | |
| *SD =standard deviation; DTZ = diltiazem hydrochloride; POL = Polycarbophil; EC4 = ethylcellulose Ethocel Standard 4 Premium Colorcon | | | | | | | | |

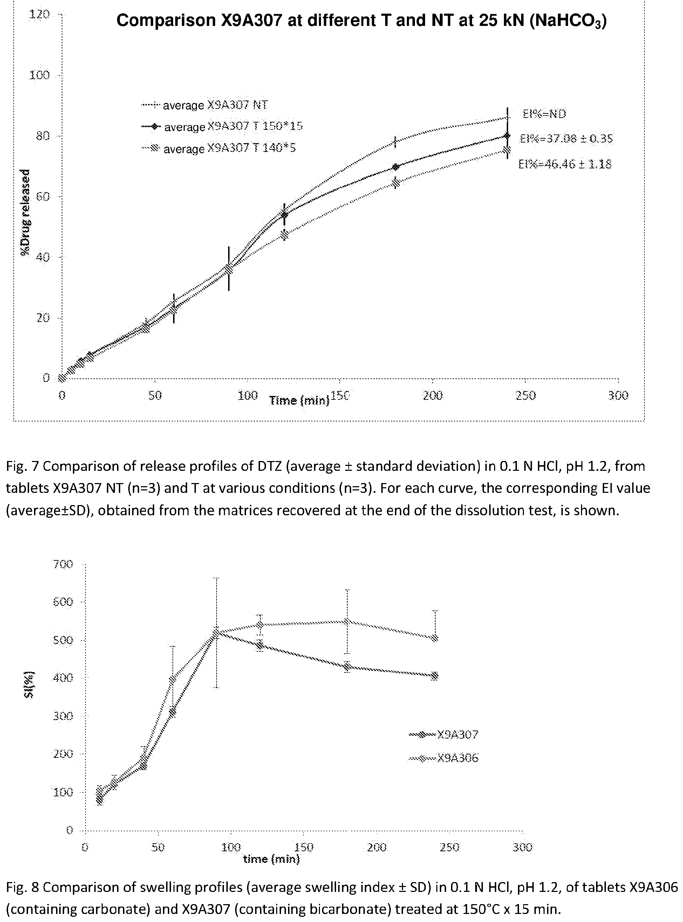

Fig. 7 shows the comparison of the release profiles of DTZ in 0.1 N HCl, pH 1.2, from cylindrical tablets subjected to a compression force of about 25 kN (X9A307) thermally treated (T) and non thermally treated (NT).

The floating behavior in 0.1 N HCl, pH 1.2, of the aforesaid tablets during the dissolution test of Fig. 7 is deduced from Table 10.

*Table 10*

| | Times (min) | | | |
|---|---|---|---|---|
| | 0 | 10 | 20 | 90 |
| X9A307 NT | FI | FI | FI | F1[10] |
| X9A307 T 140°Cx5 min | FI | FI | FI | FI |
| X9A307 T 150°Cx15 min | FI | FI | FI | FI |
| FI = floating; NF1 = not floating | | | | |

It should be noted, with reference to Fig. 8, that the swelling profile of these tablets containing sodium bicarbonate as a pH regulator is very similar to that of the tablets containing sodium carbonate. Within about 50 minutes from the start of the dissolution test, both tablets reach twice as much as their initial weight (after heat treatment) and after 100 minutes five times as much as their initial weight, which value stays almost constant and, in any case, does not decrease to values lower than 400%. Unlike the tablets containing sodium carbonate, in the case of X9A307 all the thermally treated compacts immediately float and maintain buoyancy until the end of the test (Table 10).

Table 11 shows the erosion index (EI) of the aforesaid tablets recovered at the end of the dissolution test of Fig. 7.

*Table 11*

| | X9A307 NT | X9A307 140°C × 5 min | X9A307 150°C × 15 min |
|---|---|---|---|
| EI% (average±SD) | Not determinable | 46.5±1.2 | 37.1±0.3 |

The heat treatment gives the matrix a high consistency, as is evident from the low values of the erodibility index shown in Tab. 11. The release profile of the drug is modified by the heat treatment and, as shown in Fig. 7, the release which comes closer to a zero-order kinetics is that of the compacts treated at 140°C × 5 min.

EXAMPLE 5

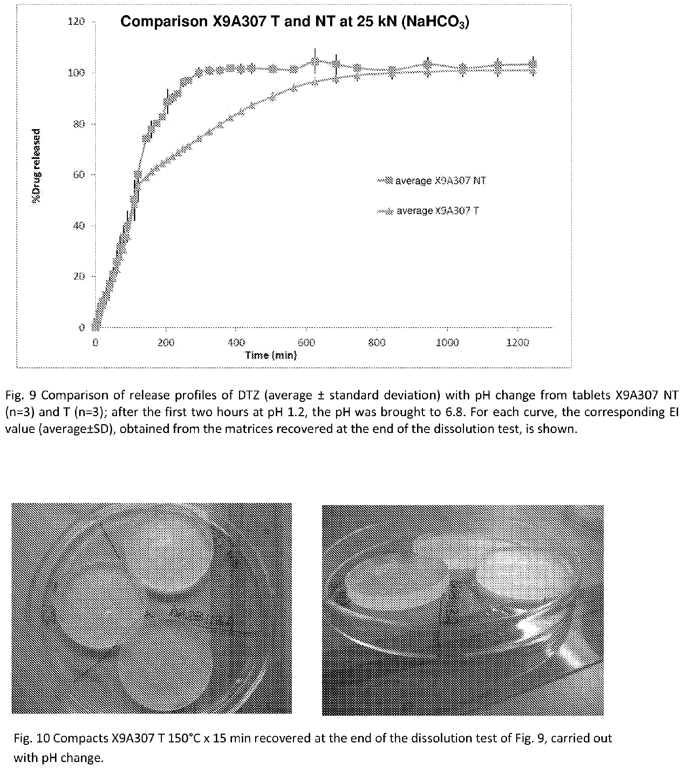

The tablets prepared in Example 4 were subjected to a dissolution test performed with pH change after 2 hours (compendial method), as illustrated in Fig. 9, to simulate the pH change due to gastrointestinal passage. Said study confirmed that the release profiles of T and NT tablets are not distinguishable until 100-120 min; afterwards, the release profile of NT becomes faster and the release from these forms is completed in about 4 hours, of which 2 hours at pH 6.8. The dissolution profile of the T forms shows that they can control the release of the drug also at pH 6.8, with a slowdown of the release rate. Under said conditions, the T forms are still capable of releasing 30-40 % of residual drug in the 10 hours following the pH change.

In fact, the NT compacts completely disintegrate after 3 h, whereas the Ts are recovered completely intact and with a very homogeneous matrix, as can be observed in the photographs of Fig. 10. The diameter of the recovered tablets is about 4 cm, with an increase, compared to the initial diameter of the tablets, of more than 200%.

EXAMPLE 6

Tablets containing disodium hydrogen phosphate ($Na_2HPO_4$) as a pH regulator were prepared according to the composition shown in Table 12.

*Table 12*

| Batch | Theoretical compression force (kN) | Code mixture | Layer composition (% w/w) | | | | Measured average compression force (kN$\pm$SD) |
|---|---|---|---|---|---|---|---|
| W9A018 | 25 | T290113B | DTZ | $Na_2HPO_4 \cdot 2H_2O$ | POL | EC4 | 24.1$\pm$1.2 |
| | | | 20 | 30 | 45 | 5 | |
| *SD =standard deviation; DTZ = diltiazem hydrochloride; POL = Polycarbophil; EC4 = ethylcellulose Ethocel Standard 4 Premium Colorcon | | | | | | | |

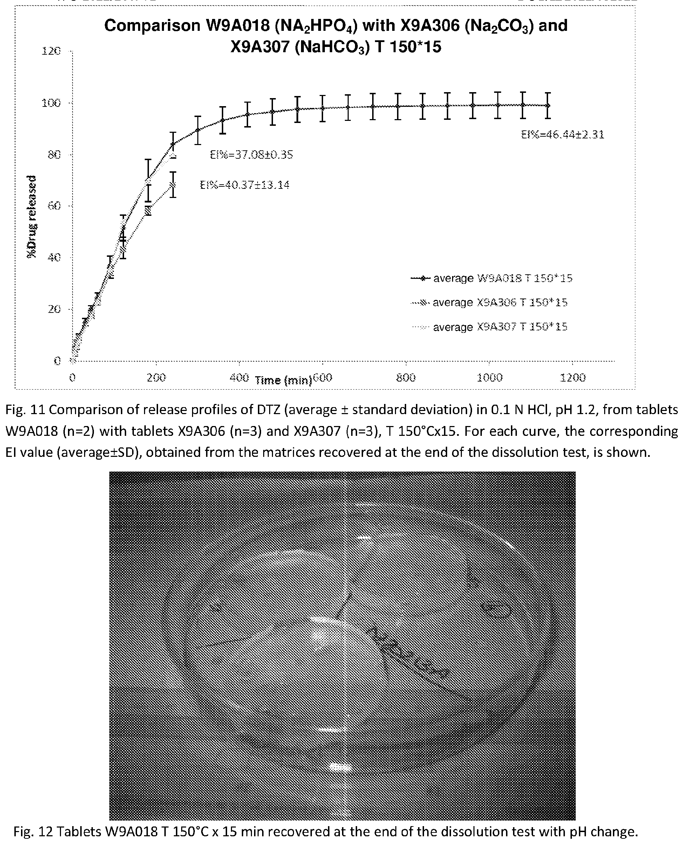

Fig. 11 shows the comparison of the release profiles of DTZ in 0.1 N HCl, pH 1.2, of W9A018 tablets (n=2) with X9A306 (n=3) and X9A307 (n=3) tablets according to examples 1 and 4, all thermally treated at 150°C for 15 minutes.
Also in the case of W9A018 tablets, the pH regulator (disodium hydrogen phosphate) after heat treatment makes the matrix swelling in acidic environment and capable of controlling the release, as in the case of compacts containing other alkaline buffering agents with which the release is compared. The W9A018 matrices considerably increase their size (Fig. 12) although they do not float. However, the choice of this excipient may be an acceptable alternative in the case that the formulation contains substances incompatible with carbonates or when there is no need for a floating form. For the purposes of gastroretention, the considerable size increase compensates for the fact that said tablets do not float, moreover, the high density, by promoting deposition of the system on the bottom of the stomach, prevents its expulsion into the intestinal tract, thereby allowing its retention.
Erosion is poor too, as can be observed in Fig. 12.

EXAMPLE 7

This example shows the results obtained with tablets containing baclofen as the active substance and sodium bicarbonate or sodium carbonate as pH regulators, according to the composition shown in Table 13.

*Table 13*

| Batch | Theoretical compression force (kN) | Tablet composition (% w/w) | | | | Measured average compression force (kN$\pm$SD) | Average weight (mg $\pm$SD) |
|---|---|---|---|---|---|---|---|
| X9A308 | 25 | Baclofen | $Na_2CO_3$ | POL | EC4 | 25.8$\pm$0.7 | 303.0$\pm$1.4 |
| | | 10 | 30 | 45 | 15 | | |
| | | | | | | | |
| X9A309 | 25 | Baclofen | $NaHCO_3$ | POL | EC4 | 25.0$\pm$0.6 | 302.1$\pm$1.5 |
| | | 10 | 30 | 45 | 15 | | |
| *SD =standard deviation; POL = Polycarbophil; EC4 = ethylcellulose Ethocel Standard 4 Premium Colorcon | | | | | | | |

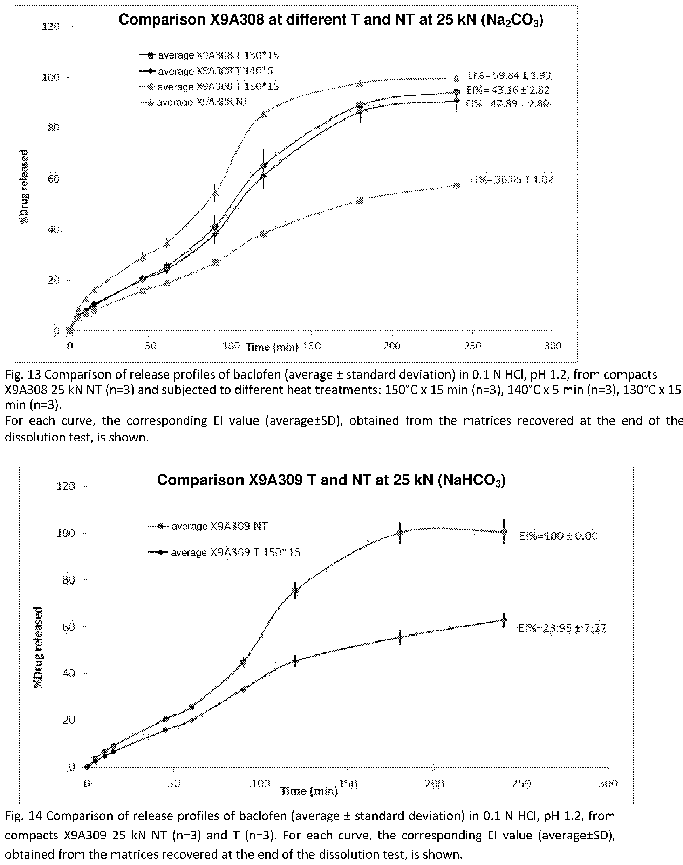

Both tablets containing, as a pH regulator, sodium carbonate (Fig. 13) and those containing sodium bicarbonate (Fig. 14) show after heat treatment the capability of swelling in acidic environment, of controlling the release of the drug they contain, and, even if their size increases, of resisting erosion. Moreover, the properties involved in gastroretention can be adjusted using different heat treatments. Furthermore, the heat treatment makes these matrices float immediately or within 10 min from the start of the test, as can be observed in Table 14.

*Table 14*

|  | Times (min) | | | |
|---|---|---|---|---|
|  | **0** | **10** | **20** | **90** |
| **X9A308 NT** | NF1 | NFI | FI | FI |
| **X9A308 T 140°Cx5 min** | F1 | FI | FI | FI |
| **X9A308 T 130°C×15 min** | F1 | FI | FI | FI |
| **X9A308 T 150°Cx15 min** | NF1 | FI | FI | FI |
| **X9A309 NT** | F1 | FI | FI | FI |
| **X9A309 T 150°Cx15 min** | F1 | FI | FI | FI |
| FI = floating; NF1 = not floating | | | | |

Table 15 shows a comparison of the erosion indexes (EI) between X9A308 and X9A309 tablets with or without heat treatment, recovered at the end of the dissolution test of Fig. 13 and 14.

*Table 15*

|  | **X9A308 NT** | **X9A308 T 150°Cx15 min** |
|---|---|---|
| **EI% (average±SD)** | 59.9±1.9 | 36.1±1.0 |
|  | **X9A309 NT** | **X9A309 T 150°Cx15 min** |
| **EI% (average±SD)** | 100 | 23.9±7.3 |

In both tablet types, with sodium carbonate and with sodium bicarbonate, the heat treatment generates a matrix which swells in the gastric fluid, capable of controlling the release of baclofen, slowing it down, until approximating a zero-order kinetics with treatments at 150°C for 15 min (Fig. 13 and 14). By varying the heat treatments, it is possible to adjust the different properties involved in gastroretention (Fig. 13). It should be noted that the heat treatment makes both matrices less erodible compared to non thermally treated compacts (Tab. 15). In particular, matrices containing sodium bicarbonate double their size after 60 min and at the end of the dissolution test are intact, since their dry residual weight is almost equal to that of the insoluble portion of the tablet formulation, whereas NTs completely disintegrate within the first 4 hours. This matrix floats immediately, like those containing sodium carbonate, except for those treated at 150°C for 15 min, which float after 10 min from the start of the dissolution test.

EXAMPLE 8

In this example, for the purpose of modulating the rate of drug release from the matrices, bilayer and tri-layer tablets with at least one layer made according to the teaching of EP 2 344 140 B1, wherein layers containing at least one pH regulator according to the present invention were added to said at least layer, were produced. DTZ was used as a model drug.

The compositions of the different layers are shown in Table 16.

*Table 16*

| Batch | Tablet type | Theoretical compr. force (kN) | Drug dose (mg) | Layer composition (%, w/w) | | | | Measured average compr. force (kN ±SD) | Average weight (mg±SD) |
|---|---|---|---|---|---|---|---|---|---|
| **W9A015** | bilayer | 25 | 60 | **DTZ** | **NaHCO$_3$** | **POL** | **EC4** |  |  |
|  | 1st layer |  |  | 20 | 30 | 45 | 5 |  |  |

(continued)

| Batch | Tablet type | Theoretical compr. force (kN) | Drug dose (mg) | Layer composition (%, w/w) | | | | Measured average compr. force (kN ±SD) | Average weight (mg±SD) |
|---|---|---|---|---|---|---|---|---|---|
| | | | | DTZ | Super-disinteg rating agent | | | | |
| | 2nd layer | | | 10 | 90 | | | 23.5±1.2 | 404.4±0.7 |
| | | | | | | | | | |
| W9A016 | bilayer | 25 | 40 | DTZ | NaHCO₃ | POL | EC4 | | |
| | 1st layer | | | 20 | 30 | 45 | 5 | | |
| | | | | DTZ | NaHCO₃ | POL | EC4 | | |
| | 2nd layer | | | 0 | 40 | 55 | 5 | 24.8±1.1 | 404.9±0.6 |
| | | | | | | | | | |
| W9A017 | tri-layer | 25 | 66.5 | DTZ | NaHCO₃ | POL | EC4 | | |
| | 1st layer | | | 20 | 30 | 45 | 5 | | |
| | | | | DTZ | MicroceLac | POL | EC4 | | |
| | 2nd layer | | | 20 | 30 | 30 | 20 | | |
| | | | | DTZ | Super-disinteg rating agent | | | | |
| | 3rd layer | | | 10 | 90 | | | 27.4±2.2 | 409.7±0.6 |

*SD =standard deviation; DTZ = diltiazem hydrochloride; POL = Polycarbophil; EC4 = ethylcellulose Ethocel Standard 4 Premium Colorcon

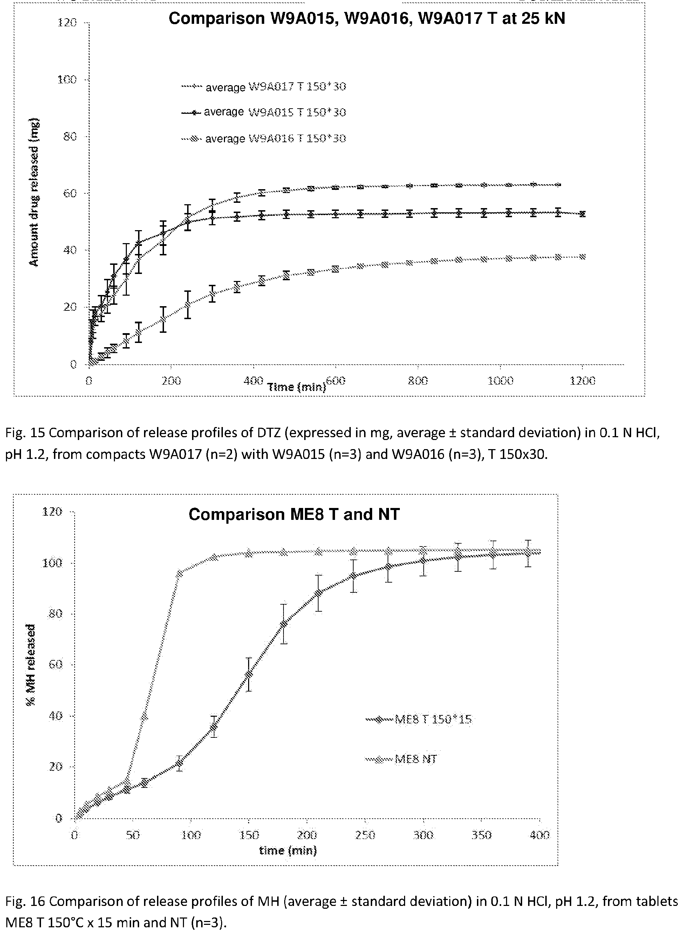

All bilayer compacts have a layer containing sodium bicarbonate, DTZ, Polycarbophil and ethylcellulose, aimed at obtaining a floating tablet, and said layer is associated in one batch (W9A015) to a layer containing DTZ and a super-disintegrating agent, for the purpose of producing an immediate drug release, whereas in another bilayer compact (W9A016) a layer contains sodium bicarbonate, Polycarbophil and ethylcellulose and is free of active substance. Finally, the tri-layer compact (W9A017) consists of a base layer with sodium bicarbonate, DTZ, Polycarbophil and ethylcellulose, which is the same layer that bilayer compacts have, an intermediate layer with a composition according to EP 2 344 140 B1, and a third layer containing DTZ and a super-disintegrating agent. All multilayer tablets were subjected to heat treatment at 150°C for 30 min and proved to be able to control the release of the drug in acidic environment (Fig. 15). The compacts that control drug release for the longest time are the bilayer compacts without super-disintegrating agent. The forms W9A015 and W9A017 float immediately, form W9A016 after 10 min.

EXAMPLE 9

This example describes coated tablets or tablets of the *core-tablet* type containing, as model drug, 70 mg metformin hydrochloride (MH), 60 mg of which being in the core. The bicarbonate is in both the coating and the core, although in a smaller amount. These tablets release the whole drug within about 5 hours with a release kinetics which, in the central part, approximates a zero-order kinetics, remaining floating for not less than 25 h. At the end of the dissolution test, they are more intact than NTs, considering the loss of the drug and the soluble fraction of the matrix, and swollen with an average diameter increase higher than 60 %.
The cores of said tablet, called ME8, were obtained by applying a compression force of 32 kN to about 93 mg powders, thereby obtaining cylindrical compacts of 7-mm diameter, to which the coating was applied by compression (50 kN for 1 minute), thereby obtaining a cylindrical tablet of 10-mm diameter and a total weight of 270 mg (Tab. 17).

Table 17

| Percentage composition of tablet ME8 | | |
|---|---|---|
| ME8 | Core mg 93 (32 kN-7 mm) | Coating mg 173 (50 kN-10 mm) |
| MH | 60.0 | 10.00 |
| POL | 11.2 | 34.68 |
| EC4 | 24.4 | 38.83 |
| BIC | 4.4 | 16.49 |
| BIC = sodium bicarbonate | | |

From Fig. 16 is clear, that this matrix, subjected to heat treatment, can control MH release also in an acidic environment. The thermally treated (T) tablet is capable of floating immediately, maintaining this property for the whole duration of the dissolution test, i.e., 25 h, whereas NTs float after 20 minutes. Moreover, as shown in Table 18, Ts and NTs display significant differences in both swelling and erosion. In fact, Ts swell definitely more, remaining intact, considering the released drug and the soluble fraction of the matrix which is lost during the dissolution test, until the end of the dissolution test.

Table 18

| | SI% (average±SD) | EI% (average±SD) |
|---|---|---|
| ME8 T 150°Cx15 min | 416.5 ± 10.2 | 40.4 ± 0.8 |
| ME8 NT* | 345.9 ± 8.2 | 54.3 ± **10.1** |
| *disintegrated | | |

EXAMPLE 10

With *core-tablets* it is possible to produce formulations (Table 19) like those containing metformin described in the present example, which, when they are co-ingested or co-administered (for example two or more tablets in one capsule) allow a prolonged coverage of the release of the drug in a therapeutically effective and safe amount.

Table 19

| Percentage composition (w/w) and morphological characteristics of tablets ME9, ME10, ME11. | | |
|---|---|---|
| ME9 | Core 93 mg (32 kN-7 mm) | Coating 807 mg (50 kN-13 mm) |
| MH | 60.0 | 1.5 |
| POL | 11.2 | 38.0 |
| EC4 | 24.4 | 42.5 |
| BIC | 4.4 | 18.0 |
| | | |
| ME10 | Core 100 mg (50 kN-10 mm) | Coating 800 mg (30 kN-13 mm) |
| MH | 60.0 | 1.5 |
| POL | 11.2 | 38.0 |
| EC4 | 24.4 | 42.5 |
| BIC | 4.4 | 18.0 |
| | | |

(continued)

| ME11 | Core 150 mg (50 kN-10 mm) | Coating 800 mg (30 kN-13 mm) |
|---|---|---|
| MH | 60.0 | 1.5 |
| POL | 11.2 | 38.0 |
| EC4 | 24.4 | 42.5 |
| BIC | 4.4 | 18.0 |

For ME9, the cores (having 93-mg weight, 7-mm diameter and obtained with a 32-kN force) were applied a coating of about 800 mg with a 50-kN compression force for one minute, thereby obtaining coated tablets of 13-mm diameter and final weight equal to about 900 mg.

The cores of 10-mm diameter of the ME10 and ME11 formulations were obtained by compressing at 30 kN 100 mg and 150 mg premixed powder, respectively. The cores were centered between two coating layers and compressed at 30 kN for 1 minute. The final compacts had a 13-mm diameter and a 900-mg (ME10) and a 950-mg weight (ME11).

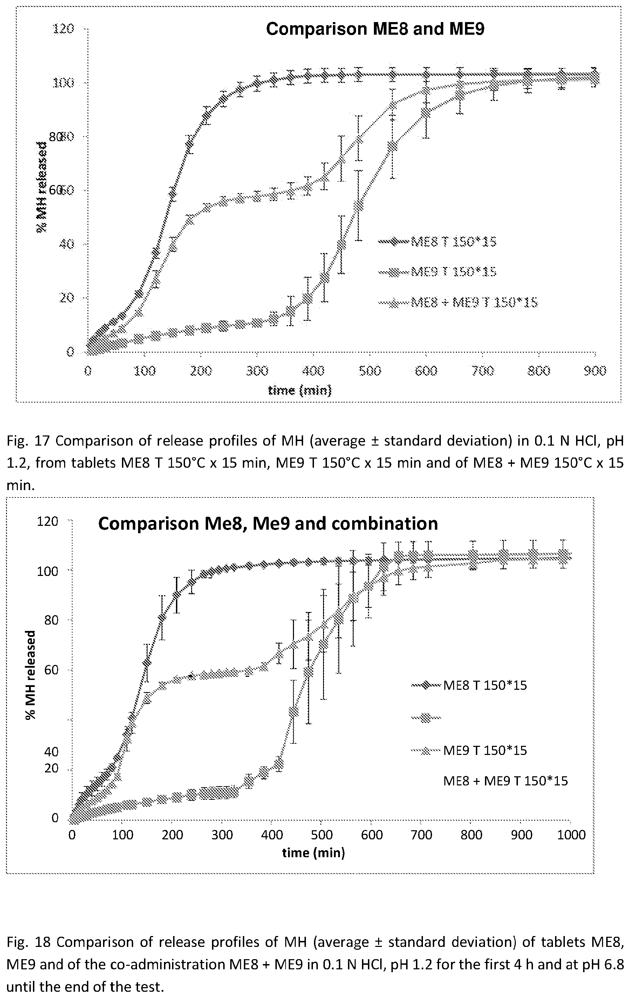

By differently modulating the start time of the second release, it is possible to obtain a release at intervals or "pulsatile" release of the active ingredient, which is useful in certain therapeutic treatments, for example to avoid saturations or downregulation of intestinal transport carriers. In fact, reducing the amount of coating allows to reduce the *lag-time* of the release (cf. ME8 in Fig. 17). Increasing the amount of coating and changing its composition allows to increase the *lag-time* by more than 6 h (cf. ME9 in Fig. 17), although still maintaining matrix buoyancy (Tab. 20).

Table 20

| Floating times of tablets ME9, ME10 and ME11 | | | | | |
|---|---|---|---|---|---|
| | t0 | t20 | t360 | t420 | t1500 |
| ME9 T 150°Cx15 min | F1 | F1 | F1 | NF1 | NF1 |
| ME10 T 150°Cx15 min | FI | FI | FI | FI | FI |
| ME11 T 150°Cx15 min | FI | FI | FI | FI | FI |

The association of these two formulations, in a combined administration, allows to obtain a total drug release of pulsated type, with the release of two doses within about 10 h, separated by a non-release time of about 200 min (see curve sum of ME8+ME9 T 150*15 in Fig. 17).

Moreover, performing a dissolution test with pH change at the fourth hour (considered as the minimum residence time in the gastric environment), it was investigated whether the release profile of the drug released by ME9 could be influenced by a sudden pH change due to an unexpected and earlier passage through the pylorus. This study showed that form ME9 could continue to control the release, in the same way, even when the pH changed to intestinal pH conditions (Fig. 18). Moreover, it is worth noting that tablets T recovered at the end of the DT had a cylindrical shape, although greatly swollen (diameter of 32.78 $\pm$ 0.6 mm, n=6), whereas the same tablets NT were completely fragmented.

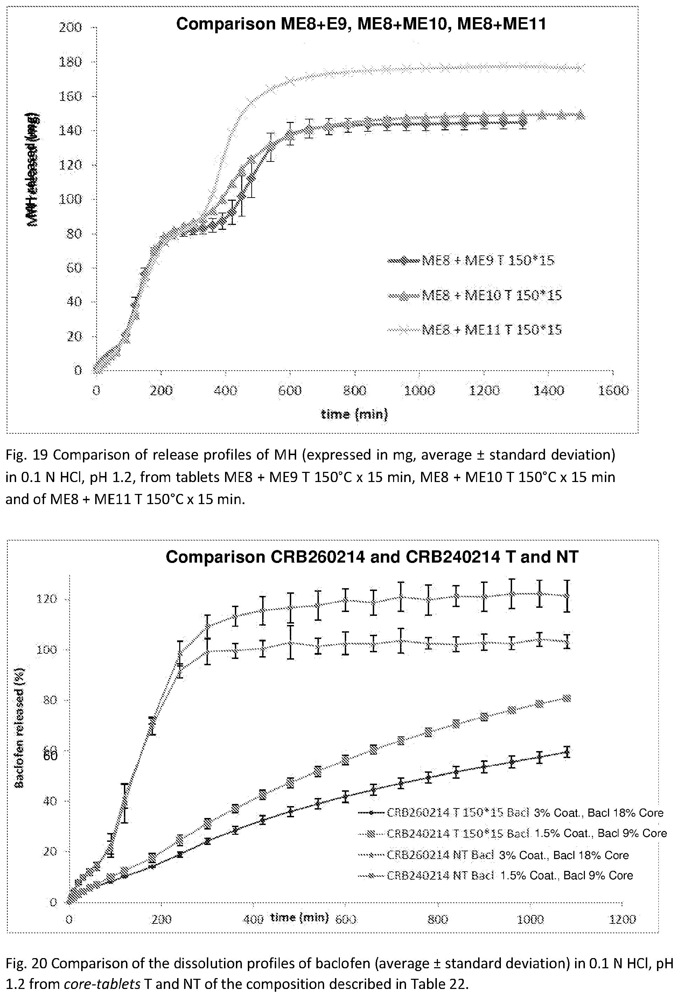

Moreover, it is clear from the comparison in Fig. 19, that with shrewd modifications of the compaction forces, of the weight of the core and/or of the coating, as well as of the ratio between core weight and coating weight, one can influence the start time and/or the rate of the release of the active ingredient, the floating time and the swelling of the form in contact with the fluid simulating gastric pH. This allows to design a pharmaceutical form with characteristics of release start, release rate, loaded dose, swelling and buoyancy which are the most suitable for the therapeutic needs of the active ingredient used. The ratio between coating weight and core weight ranges, in the release profiles described in Fig. 19, between 1.86 (ME8) and 8.68 (ME9). ME10 and ME11 have the same *lag-time* of release start, but the release rate for ME11 is clearly higher compared to ME10. Instead, ME9 (93-mg core) has the longest *lag-time* and a release rate higher than ME10 (100-mg core), but lower than ME11 (150 mg). These three formulations have the same percentage composition, but different weight ratios between core and coating, as well as different compaction forces. Different core size (for example from 7 to 10 mm) and different compression forces are used to obtain core and coating (from 30 to 50 kN) and different behavior of swelling and erosion; in any case, also the average diameter of the forms recovered after DT is clearly increased and, in the case of formulations M9, M10 and M11, is higher than 20 mm, reaching even 25 mm (tab. 21).

*Table 21*

| Swelling index and erosion index (average±SD) of 4 core-tablets formulations | | | |
|---|---|---|---|
| | **SI% (average±SD)** | **EI% (average±SD)** | **Average diameter (mm) at the end of the DT (average±SD)** |
| **ME8 T 150°Cx15 min** | 416.5 ± 10.2 | 40.4 ± 0.8 | 17.3 ± 0.4 |
| **ME9 T 150°Cx15 min** | 645.8 ± 21.4 | 24.6 ± 1.3 | 24.6 ± 0.4 |
| **ME10 T 150°Cx15 min** | 594.0 ± 4.30 | 23.5 ± **1.1** | 24.2 ± 0.5 |
| **ME11 T 150°Cx15 min** | 583.3 ± **12.1** | 25.2 ± 0.1 | 24.3 ± 0.6 |

EXAMPLE 11

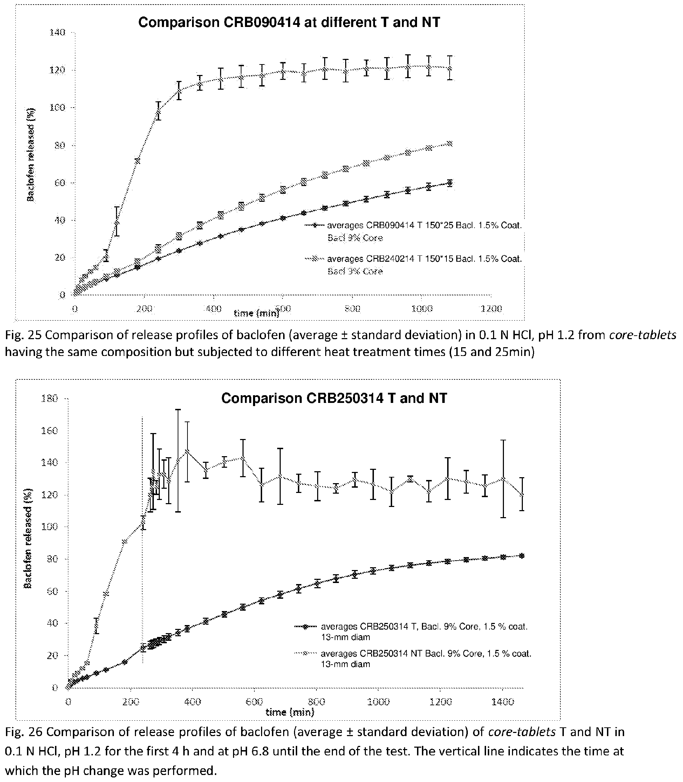

This example describes different types of core-tablets containing baclofen or baclofen hydrochloride; as shown in Fig. 25, the tablets have a different release profile depending on the different heat treatment.

The different types of tablets differ in the amount of baclofen in the core and in the coating or differ in the weight of the core or of the coating, as can be deduced from Tables 22 and 23.

*Table 22*

| Composition of core-tab tablets containing baclofen | | | | | |
|---|---|---|---|---|---|
| **CRB060214B** | | | | | |
| | | **POL%** | **NaHCO$_3$%** | **EC4%** | **BAC%** |
| **MB210114** | core | 25.0 | 10.0 | 55.0 | 10.0 |
| **MRB040214B** | coat. | 70.0 | 30.0 | 0 | 0 |
| | | | | | |
| **CRB110214B** | | | | | |
| | | **POL%** | **NaHCOs%** | **EC4%** | **BAC%** |
| **MB290114** | core | 19.39 | 0 | 70.61 | 10 |
| **MRB100214** | coat. | 40.0 | 16.0 | 43.0 | 1.0 |

| **CRB240214** | | | | | |
|---|---|---|---|---|---|
| | | **POL%** | **NaHCO$_3$%** | **EC4%** | **BAC%** |
| **MB210214** | core | 25 | 10 | 56 | 9 |
| **MRB190214** | coat. | 40 | 16 | 42.5 | 1.5 |
| | | | | | |
| **CRB260214** | | | | | |
| | | **POL%** | **NaHCOs%** | **EC4%** | **BAC%** |
| **MB250214** | core | 22 | 9 | 51 | 18 |
| **MRB250214** | coat. | 39.4 | 15.8 | 41.9 | 3 |
| | | | | | |
| **CRB050314** | | | | | |
| | | **POL%** | **NaHCO$_3$%** | **EC4%** | **BAC%** |
| **MB250214** | core | 22 | 9 | 51 | 18 |
| **MRB250214** | coat. | 39.4 | 15.8 | 41.9 | 3 |

(continued)

| CRB110314 | | POL% | NaHCO$_3$% | EC4% | BAC% |
|---|---|---|---|---|---|
| **MB100314** | core | 19.40 | 7.94 | 44.96 | 27.70 |
| **MRB100314** | coat. | 38.58 | 15.44 | 40.98 | 5.00 |

The cylinder-shaped tablets contain a core, obtained by applying a 12-kN compression force, which is cylindrical and has a 7- or 9-mm diameter and respective average weight of about 90 or 150 mg. The coating having average weight of about 180 (for a core of 7-mm diameter) or 300 (for a core of 9-mm diameter) mg was applied with a force of about 50 kN to obtain a *core-tablet* with a 10- or 13 mm diameter and a total weight of about 270 or 450 mg (Table 23).

Table 23

| | Core diameter (mm) | Core average weight (mg) | CT diameter(mm) | CT average weight (mg) |
|---|---|---|---|---|
| **CRB060214B** | 9 | 150 | 13 | 450 |
| **CRB110214B** | 9 | 150 | 13 | 450 |
| **CRB240214** | 9 | 150 | 13 | 450 |
| **CRB260214** | 9 | 150 | 13 | 450 |
| **CRB050314** | 7 | 90 | 10 | 280 |
| **CRB110314** | 7 | 90 | 10 | 270 |
| CT = coated tablet | | | | |

As expected, thermally treated tablets have a very different drug release profile compared to NT tablets, and it is worth noting that the matrix gains the capability to control drug release also in acidic environment (pH 1.2), producing a release kinetics of the drug comparable to a zero order (Fig. 20).

The core-tablets compared in Fig. 20 have the same qualitative composition of the core and of the coating, but one of the two formulations contains double the percentage amount of baclofen contained in the other one, both in the core and in the coating (Tab. 22). The example shows that by doubling the drug amount the release kinetics does not change and is still comparable to a zero-order kinetics.

Moreover, during the dissolution test in acidic environment (pH = 1.2), the tablets T swell and float immediately and for the whole duration of the test (even up to 24 h). The tablets, recovered at the end of the test, show a very high swelling (SI > 500 %) and are almost intact (average EI about 14%) (Table 24).

Table 24

| Swelling index (SI) and erosion index (EI) of core-tablets CRB060214B, CRB110214B, CRB240214, CRB 260214, CRB050314 and CRB110314 | | | |
|---|---|---|---|
| | | **SI(%)±SD** | **EI(%)±SD** |
| **CRB060214B** | **core+coat.** | 716.60±12.3 | 13.3±0.8 |
| **CRB110214B** | **core+coat.** | 484.05±44.3 | 7.6±0.6 |
| **CRB240214** | **core+coat.** | 538.80±22.4 | 13.9±0.7 |
| **CRB260214** | **core+coat.** | 595.34±14.1 | 14.3±0.1 |
| **CRB050314** | **core+coat.** | 462.99±25.2 | 16.9±0.1 |
| **CRB110314** | **core+coat.** | 326.26±25.7 | 10.3±1.3 |

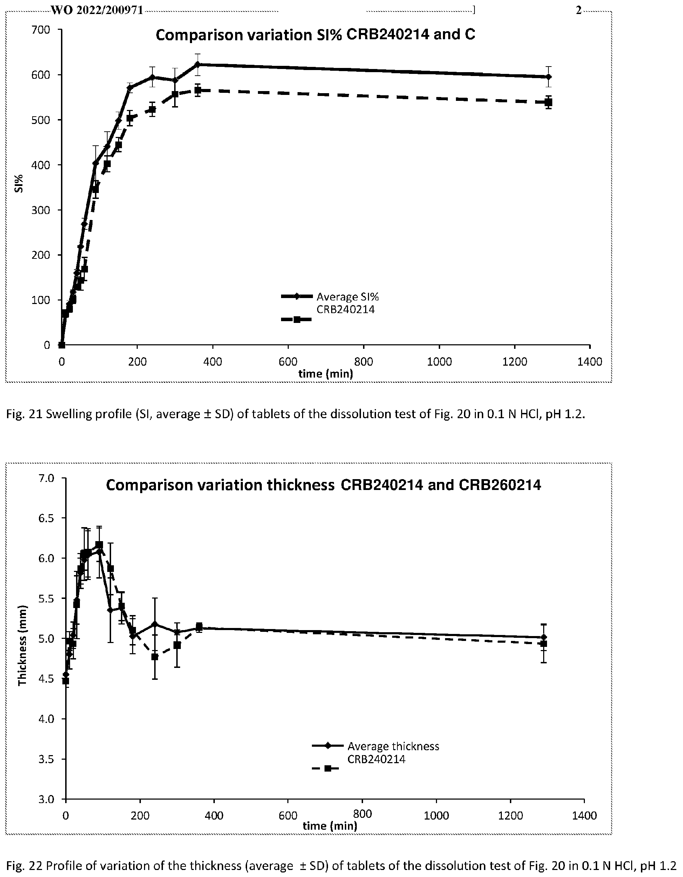

As shown in Fig. 21, 22 and 23, for the core-tablets of composition described in Tab. 22, the profiles of SI, thickness variation and diameter variation during the dissolution test in acidic environment are correlated for the two types of tablets and are therefore scarcely influenced by the amount of drug loaded in the pharmaceutical form.

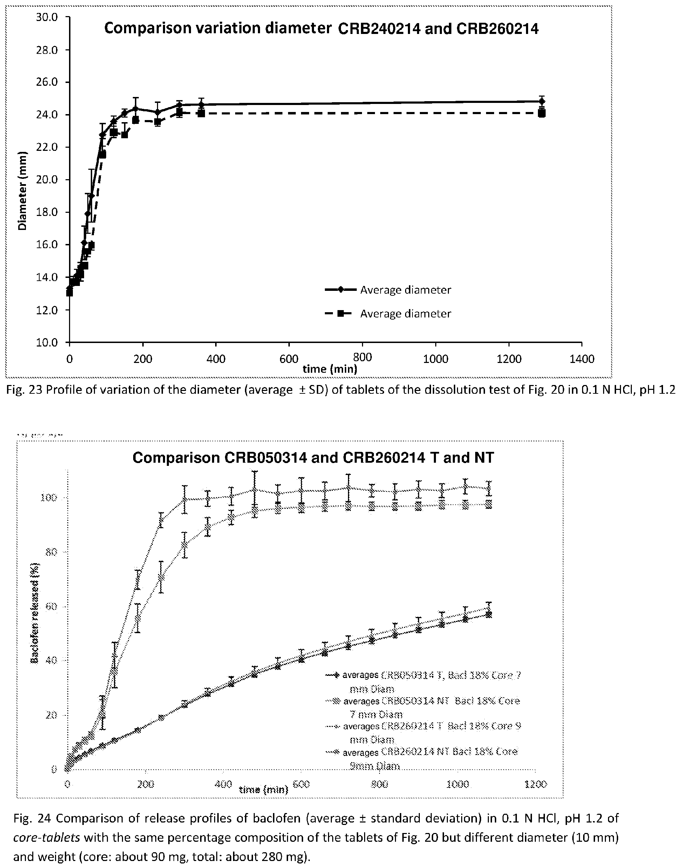

The average diameter larger than 20 mm (average increase of 54% of the diameter compared to the initial diameter) is reached between 60 min and 90 min for both formulations (Fig. 23).

Fig. 24 shows the release profile of tablets having the same composition as CRB260214 (Tab. 22) but different size (10 mm versus 13 mm, Tab. 23), and it can be noted that the decrease of size of core-tablets of identical percentage composition does not change the rate of drug release.

Table 25

| CRB090414 (core 9 mm, total diameter 13 mm) | | POL% | NaHCO$_3$% | EC4% | BAC% |
|---|---|---|---|---|---|
| MB210214 | core | 25 | 10 | 56 | 9 |
| MRB310314 | coat. | 40 | 16 | 42.5 | 1.5 |

Fig. 25 shows that different times of heat treatment of baclofen-containing core-tablets with composition as in Table 25 (identical to that of CRB240214) allow to modulate the rate of drug release.

Instead, Fig. 26, with a dissolution test with pH change after 4 h, shows that the release of the drug continues to have a zero-order kinetics even at intestinal pH. The composition of the core-tablets of Fig. 26 is shown in Table 26. SI and EI of core-tablets CRB090414 and CRB250314 are shown in Tab. 35 and 36 of example 17.

Table 26

| CRB250314 (core 9 mm, total diameter 13 mm) | | POL% | NaHCO$_3$% | EC4% | BAC% |
|---|---|---|---|---|---|
| MB210314 | core | 25 | 10 | 56 | 9 |
| MRB190214 | coat. | 40 | 16 | 42.5 | 1.5 |

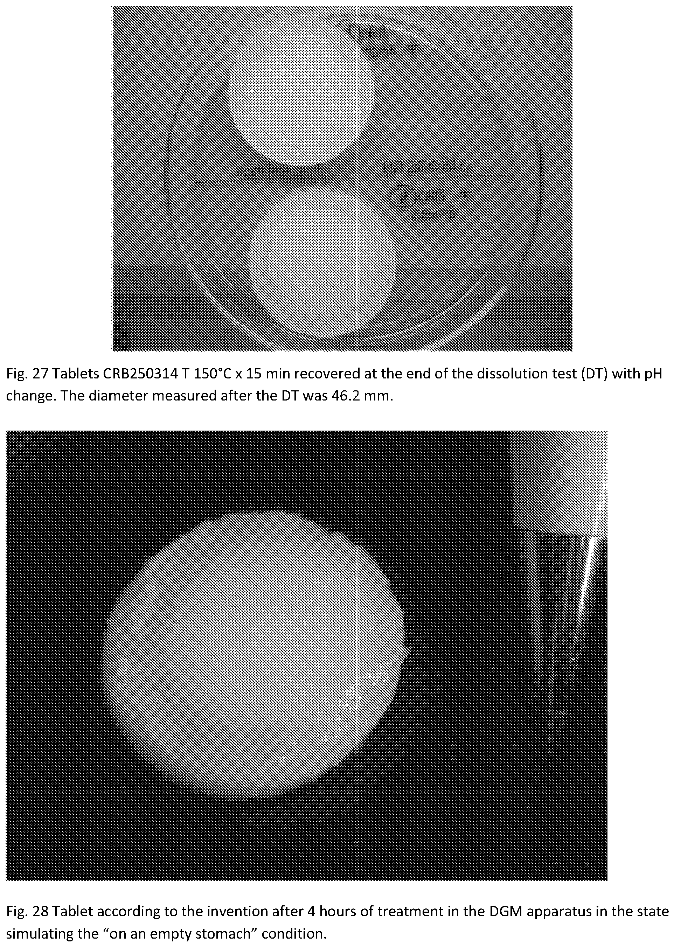

Fig. 27 shows clearly that the tablets after 24h dissolution test are intact and swollen (SI higher than 3000%), reaching a diameter of 46 mm.

EXAMPLE 12

In example 12, to evaluate the floating behavior of the dosage forms in the stomach, the time of passage and the integrity of the dosage form after said passage, an apparatus called DGM was used, which is a patented apparatus simulating the behavior in the stomach (EP 1 907 108).

Tablets of the core-tablet type subjected to heat treatment of 150°C × 15 min (GR) were compared, in the DGM, with analogous tablets which were not subjected to heat treatment (CR).

The core-tablet composition is shown in Table 27.

Table 27

| Core-tablet formulation with baclofen as the model drug | | | | | | | |
|---|---|---|---|---|---|---|---|
| | Weight (mg) | Diameter (mm) | Thickness (mm) | POL% | EC4% | NaHCO$_3$% | BAC% |
| | | | | | | | |
| core | 93.4±0.2 | 7 | 2.0 | 25 | 55 | 10 | 10 |
| coating | 177.3±0.4 | | | 38 | 42.5 | 18 | 1.5 |
| core-tablet | 270.7±0.4 | 10 | 2.75 | | | | |

Firstly, 20 mL priming solution of gastric acid were introduced in the DGM, then a tablet with 240 mL tap water was added, and the DGM was set to contain only a small volume of gastric liquid, about 50 mL, after emptying. During this step, the DGM was operated in a feedback-controlled mode, wherein the speed of acid addition is controlled by the measured pH and the gastric volume sets the speed of addition of enzymatic solution. In this kind of standard experiment "on an empty stomach", the pH decreases from about 6.5 to 2. In the second step, the speed of acid addition was set to 0.9 mL/min and that of enzymatic solution to 0.1 mL/min, thereby simulating a stomach at rest and an empty stomach.

Moreover, the emptying speed was set to correspond to the speed of addition of gastric liquids, thereby maintaining the volume constant. The tests were interrupted after 4 hours or when the formulation disappeared from the surface.

For "on a full stomach" experiments, a high-calorie and fat-rich meal was used as recommended by FDA for evaluating the effects of food on the behavior of the formulation. The meal consisted of 200 g milk, two slices of toasted bread with butter, two scrambled eggs, about 200 g fries and two slices of fried bacon. The meal was chewed by a volunteer and spit in a glass container before swallowing. The chewed meal was emptied in the DGM and a tablet was introduced in the DGM together with 240 mL water. After about 3 hours, a second chewed meal consisting of 5 "digestive" biscuits (65-70 g) and 200 mL skimmed milk was added. This extended the duration of the study in the "on a full stomach" state to about 6 hours.

The integrity and buoyancy of formulations were evaluated by visually inspecting in the DGM and by recording time-lapse videos.

DGM experiments "on an empty stomach":

A difference between control formulations (CR) and gastroretentive (GR) formulations was observed. After about 2 hours treatment, the control formulation was significantly degraded or corroded. Two control sessions were ended after 2-2½ h, because the formulations had been expelled.

During the study "on an empty stomach", the gastroretentive formulation floated during the four hours, and the dosage form did not show any degradation sign after the test. At the end, it had a diameter of 21 mm and a thickness of 6 mm.

Figure 28 shows the appearance of a gastroretentive formulation after 4 hours processing "on an empty stomach".

DGM experiments "on a full stomach":

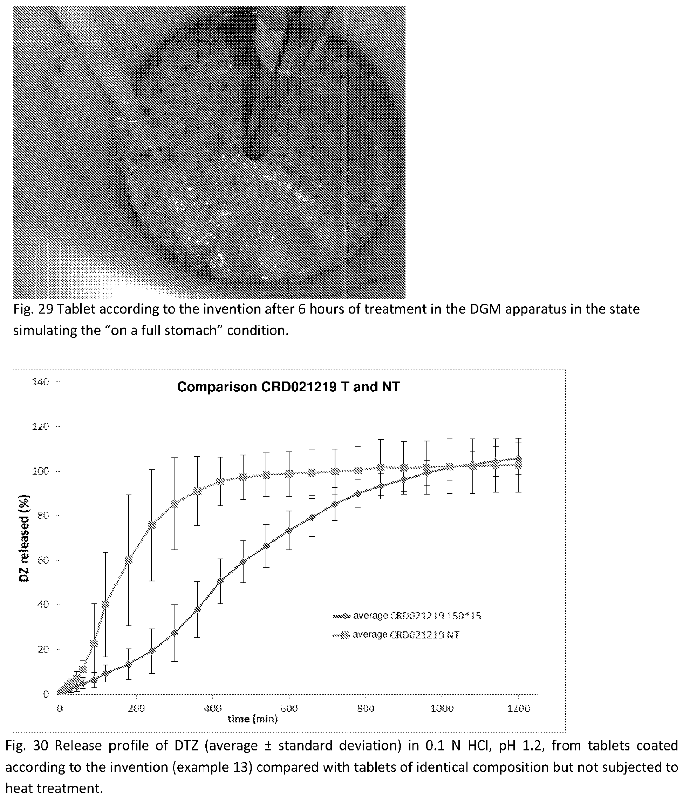

The gastroretentive formulation (GR) floated for 6 hours (Fig. 29); instead, the control formulation (CR) floated only in the first stage of the experiment, and this aspect can be correlated to a different buoyancy of the two formulations.

Conclusion:

On an empty stomach, a difference between the gastroretentive formulation (GR) and the control formulation (CR) in terms of physical integrity and buoyancy was observed. In "on a full stomach" experiments, the highest buoyancy in the gastroretentive formulation could be observed after the addition of the second meal. In neither of the three experiments the control formulation could be observed for the whole duration of the experiment, in that it disappeared from the surface after the addition of the second meal. The gastroretentive formulation could be observed for the whole duration of the experiment, and, when it was recovered after the test, it did not show any erosion signs.

EXAMPLE 13

Core-tablets containing diltiazem HCl and having a 7-mm core diameter, a 10-mm coating diameter and the composition shown in Table 28 were prepared. The tablets were subjected to heat treatment at 150°C for 15 minutes.

*Table 28*

| CRD021219 (core 7 mm, total diameter 10 mm) | | | | | |
|---|---|---|---|---|---|
| | | POL% | NaHCO$_3$% | EC4% | DTZ% |
| **MD141119** | core | 25 | 10 | 55 | 10 |
| **RD291119** | coat. | 38 | 18 | 42.5 | 1.5 |

At the end of the dissolution test shown in Fig. 30, in which the tablets thereby produced were compared with tablets that were identical but not thermally treated (NT), the tablets according to the present example had the following values of swelling index (SI) and erosion index (EI):

$$SI = 459.8 \pm 14.9\%$$

$$EI = 19.4 \pm 0.2\%$$

EXAMPLE 14

Core tablets LD5a containing levodopa and having a core with 145-mg weight and 9-mm diameter, were prepared, wherein the final tablet weighed 445 mg and had a 13-mm diameter and the composition shown in Table 29. The tablets were thermally treated at 150°C for 15 minutes.

Table 29

| LD5a | | | | | |
|---|---|---|---|---|---|
| | | POL% | NaHCO$_3$% | EC4% | LEVODOPA% |
| MD171121 | core | 25.00 | 10.00 | 55.00 | 10.00 |
| RD221121 | coat. | 34.50 | 16.50 | 39.00 | 10.00 |

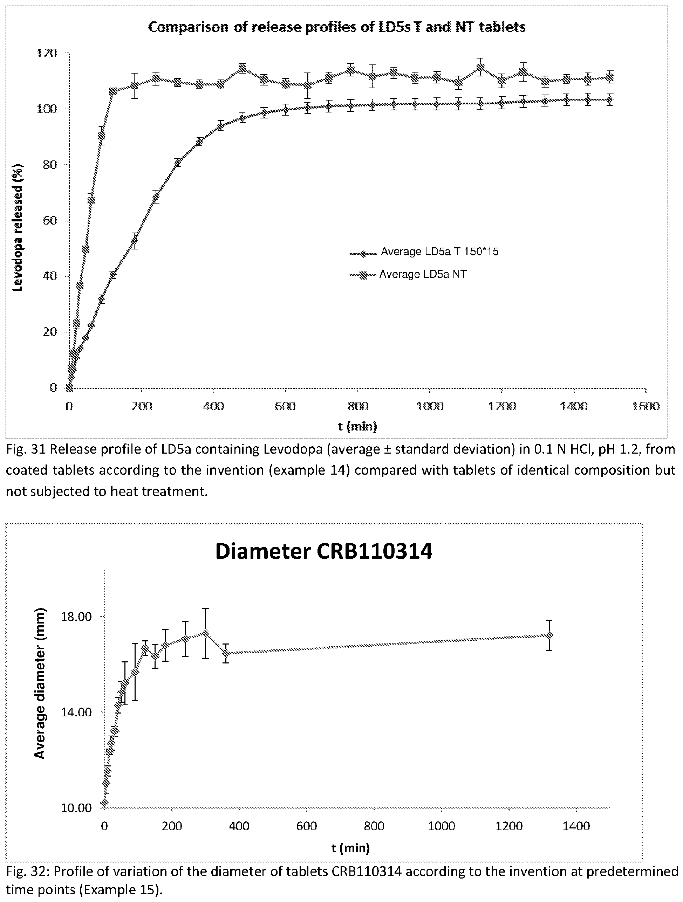

The drug release profiles of the treated tablets, compared to those of the corresponding non-treated tablets, are shown in Fig. 31.
The thermally treated tablets had a high mechanical strength, as proven by the low erosion index (EI% = 24.8 $\pm$0.2%).

EXAMPLE 15

Four baclofen core tablets CRB110314 of 10-mm diameter and 270-mg weight were prepared starting from the composition shown in the following Table 30.

Table 30

| CRB110314 | | | | | |
|---|---|---|---|---|---|
| | | POL% | NaHCO$_3$% | EC4% | BAC% |
| MB100314 | core | 19.4 | 7.94 | 44.96 | 27.7 |
| MRB100314 | coat. | 38.58 | 15.44 | 40.98 | 5 |

The tablets were treated at 150°C for 15 minutes.
The tablets thereby obtained were placed in a container containing 0.1 M HCl, pH 1.2 (1 L) at a temperature of 37°C under mild agitation at 50 rpm.
At predetermined time points (every 10 minutes during the first hour, every 30 minutes for the following two hours and afterwards every hour in the following three hours), the tablets were recovered and their weight and diameter were accurately measured. Then, the tablets were kept at 37°C under agitation for 16 additional hours. Finally, final weight and diameter were measured.
The curve of the graphic of Fig. 32 shows the diameter of the tablets at the various aforesaid time points.
The following Table 31 shows the variation of the average diameter VARDIAM%.

Table 31

| time (min) | Average VARDIAM% |
|---|---|
| 0 | 0.00 |
| 5 | 8.08 |
| 10 | 13.10 |
| 15 | 20.93 |
| 20 | 24.36 |
| 30 | 29.13 |
| 40 | 39.90 |
| 50 | 46.10 |
| 60 | 48.84 |

(continued)

| time (min) | Average VARDIAM% |
|---|---|
| 90 | 53.37 |
| 120 | 63.16 |
| 150 | 59.85 |
| 180 | 64.38 |
| 240 | 67.07 |
| 300 | 69.28 |
| 360 | 61.08 |
| 1320 | 68.54 |

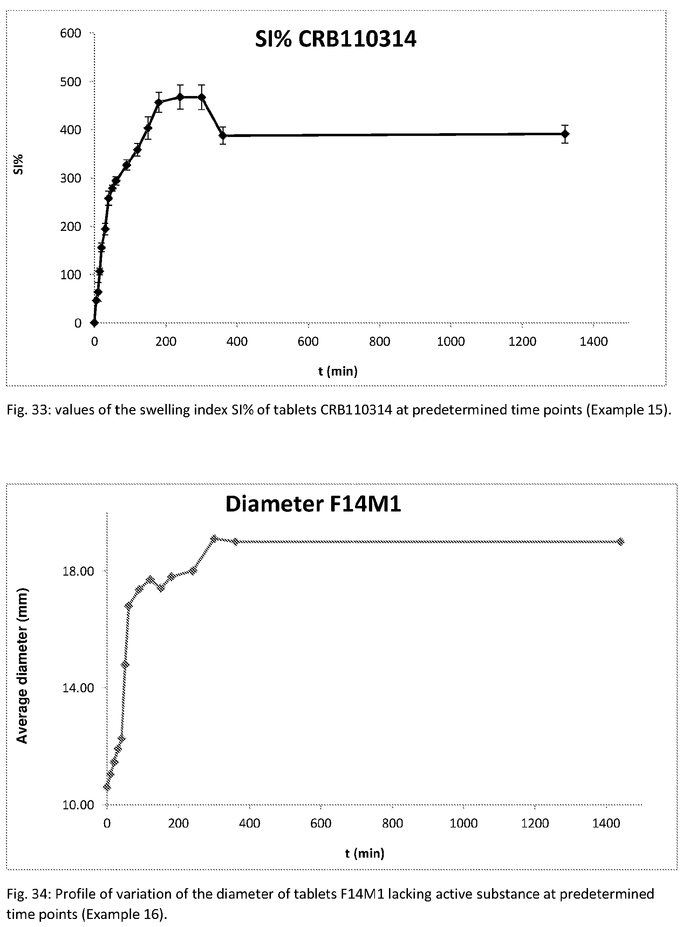

The graphic of Figure 33 shows the values of the swelling index SI% at the aforesaid time points.

EXAMPLE 16

Core tablets without active substance F14M1 of 10-mm diameter and 270-mg weight were prepared starting from the composition shown in the following Table 32.

Table 32

| F14M 1 | | | | |
|---|---|---|---|---|
| | | POL% | NaHCO$_3$% | EC4% |
| **MP030715** | core | 27.8 | 11.1 | 61.1 |
| **RP030715** | coat. | 38.6 | 18.3 | 43.1 |

The tablets were treated at 150°C for 15 minutes.
The tablets thereby obtained were placed in a container containing 0.1 M HCl, pH 1.2 (1 L) at a temperature of 37°C under mild agitation at 50 rpm.
At predetermined time points (every 10 minutes during the first hour, every 30 minutes for the following two hours and afterwards every hour in the following three hours), the tablets were recovered and their weight and diameter were accurately measured. Then, the tablets were kept at 37°C under agitation for 18 additional hours. Finally, final weight and diameter were measured.
The curve of the graphic of Fig. 34 shows the diameter of the tablets at the various aforesaid time points.
The following Table 33 shows the variation of the average diameter VARDIAM%.

Table 33

| time (min) | Average VARDIAM% |
|---|---|
| 0 | 0.00 |
| 10 | 6.71 |
| 20 | 8.02 |
| 30 | 11.83 |
| 40 | 15.57 |
| 50 | 37.10 |
| 60 | 58.49 |
| 90 | 63.98 |
| 120 | 66.98 |
| 150 | 64.15 |

(continued)

| time (min) | Average VARDIAM% |
|---|---|
| 180 | 67.92 |
| 240 | 69.81 |
| 300 | 80.19 |
| 360 | 79.25 |
| 1440 | 79.25 |

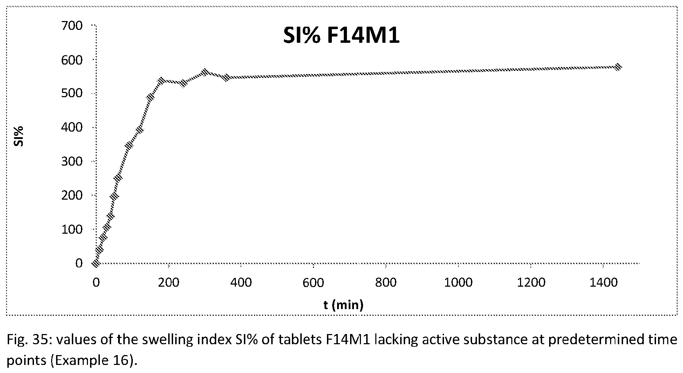

The graphic of Figure 35 shows the values of the swelling index SI% at the aforesaid time points.

EXAMPLE 17

**Determining the floating lag time and the total floating time**

Floating lag time can be defined as the time a tablet requires to emerge on the surface of a dissolution medium consisting of 0.1 M HCl at pH 1.2 (1 L) at 37°C.

The tablets described above were immersed in a container containing the abovementioned dissolution medium and the time the tablets required to reach the surface of the medium was measured by a chronometer.

The total floating time is the time during which the tablets constantly float on the surface of the abovementioned dissolution medium. Said time was determined by direct observation at 30-minute intervals and, during night, by video recording.

The following Table 34 shows the values of floating lag time and of total floating time determined for each tablet.

*Table 34*

| | Floating lag time (min) | Total floating time (min) |
|---|---|---|
| **X9A305** | 20 | 220 |
| **X9A306** | 10 | 230 |
| **X9A307** | 0 | 240 |
| **X9A308** | 10 | 20 |
| **X9A309** | 0 | 240 |
| **W9A015** | 0 | 240 |
| **W9A016** | 10 | 240 |
| **W9A017** | 0 | 240 |
| **ME8** | 0 | 1500 |
| **ME9** | 0 | 360 |
| **ME10** | 0 | 1500 |
| **ME11** | 0 | 1500 |
| **CRB060214B** | 0 | 1080 |
| **CRB110214B** | 0 | 1080 |
| **CRB240214** | 0 | 1440 |
| **CRB260214** | 0 | 1440 |
| **CRB050314** | 0 | 1080 |
| **CRB110314** | 0 | 1080 |
| **CRB250314** | 0 | 1460 |
| **CRB090414** | 0 | 1080 |
| **F14M1** | 0 | 1440 |

(continued)

|  | Floating lag time (min) | Total floating time (min) |
|---|---|---|
| CRD021219 | 0 | 1200 |
| LD5a | 0 | 1500 |

The following Table 35 summarizes the values of the swelling index (SI%) of the tablets of the above examples and the percentage variations of swelling index compared to the corresponding non-treated tablets.

Table 35

|  | SI% | VARSI% |
|---|---|---|
| X9A305 | 586.2 | 708.1 |
| X9A306 | 403.2 | 898.1 |
| X9A307 | 526.1 | NA |
| W9A018 | 489.4 | NA |
| X9A308 | 344.0 | NA |
| X9A309 | 491.6 | NA |
| W9A015 | 180.6 | NA |
| W9A016 | 504.5 | 4.4 |
| W9A017 | 227.1 | 11.4 |
| ME8 | 416.5 | NA |
| ME9 | 645.8 | NA |
| ME10 | 594.0 | NA |
| ME11 | 583.3 | 2899.2 |
| CRB060214B | 716.6 | 93.4 |
| CRB110214B | 484.0 | 112.6 |
| CRB240214 | 538.8 | 11.9 |
| CRB260214 | 595.3 | 85.9 |
| CRB050314 | 463.0 | 4.4 |
| CRB110314 | 326.3 | 23.7 |
| CRB250314 | 530.0 | NA |
| CRB090414 | 409.2 | 788.5 |
| F14M1 | 578.0 | NA |
| CRD021219 | 459.8 | NA |
| LD5a | 509.1 | NA |

The variation was calculated applying the following formula:

$$\text{VARSI\%} = (\text{average SI\%}_T - \text{average SI\%}_{NT})/\text{average SI\%}_{NT} * 100$$

wherein average $\text{SI\%}_T$ = average swelling index of tablets treated at 150°C for 15 minutes and average $\text{SI\%}_{NT}$ = average swelling index of the corresponding non-treated tablets at the end of the dissolution tests in 0.1 M HCl pH 1.2 (1 L) at 37°C.

The percentage variation of the swelling index is generally in the range between 4 and 20000, in particular in the range 10-5000.

The following Table 36 summarizes the values of the erosion index (EI%) of the tablets of the above examples and the percentage variations of erosion index compared to the corresponding non-treated tablets.

Table 36

|  | EI% | VAREI% |
|---|---|---|
| X9A305 | 47.0 | 78.7 |
| X9A306 | 40.4 | 31.7 |
| X9A307 | 37.1 | NA |
| W9A018 | 46.4 | NA |
| X9A308 | 36.1 | NA |
| X9A309 | 24.0 | NA |
| W9A015 | 71.5 | NA |
| W9A016 | 34.6 | 102.1 |
| W9A017 | 59.5 | 32.4 |
| ME8 | 40.4 | NA |
| ME9 | 24.6 | NA |
| ME10 | 23.5 | NA |
| ME11 | 25.2 | NA |
| CRB060214B | 13.3 | 361.1 |
| CRB110214B | 7.6 | 623.0 |
| CRB240214 | 13.9 | 201.2 |
| CRB260214 | 14.3 | 289.2 |
| CRB050314 | 16.9 | 116.2 |
| CRB110314 | 10.3 | 269.1 |
| CRB250314 | 13.9 | NA |
| CRB090414 | 12.4 | NA |
| F14M1 | 20.6 | NA |
| CRD021219 | 19.4 | NA |
| LD5a | 24.8 | NA |

The variation was calculated applying the following formula:

$$\mathrm{VAREI\%} = (\mathrm{average}\ EI\%_{NT} - \mathrm{average}\ EI\%_{T})/\mathrm{average}\ EI\%_{T}*100$$

wherein average $EI\%_{NT}$ = average erosion index of non-treated tablets and average $EI\%_{T}$ = average erosion index of the corresponding tablets treated at 150°C for 15 minutes at the end of the dissolution tests in 0.1 M HCl pH 1.2 (1 L) at 37°C.

The percentage variation of the erosion index is generally higher than 25 and is typically in the range from 30 to 700.

The following Table 37 summarizes the values of the diameter of the tablets at the end of the dissolution tests in 0.1 M HCl, pH 1.2 (1 L) at 37°C and shows the percentage diameter variations compared to the corresponding tablets before being subjected to the dissolution test.

Table 37

|  | DIAMETER (mm) | VARDIAM% |
|---|---|---|
| X9A305 | 13 | 84.2 |
| X9A306 | 13 | 89.5 |
| X9A307 | 13 | 87.7 |
| ME8 | 10 | 65.0 |

(continued)

|  | DIAMETER (mm) | VARDIAM% |
|---|---|---|
| ME9 | 13 | 80.4 |
| ME10 | 13 | 73.6 |
| ME11 | 13 | 74.7 |
| CRB060214B | 13 | 8.0 |
| CRB110214B | 13 | 79.8 |
| CRB240214 | 13 | 85.7 |
| CRB260214 | 13 | 84.9 |
| CRB050314 | 10 | 70.5 |
| CRB110314 | 10 | 68.5 |
| CRB090414 | 13 | 75.5 |
| F14M1 | 10 | 79.6 |
| CRD021219 | 10 | 74.3 |
| LD5a | 13 | 83.9 |

The diameter variation was calculated using the following formula:

$$VARDIAM\% = [(diam_t - diam_0)/diam_0 * 100)$$

wherein $diam_t$ = diameter of the tablets treated at 150°C for 15 minutes, measured at the end of the dissolution test, and $diam_0$ = diameter of the tablets treated at 150°C for 15 minutes measured at the end of the heat treatment.

The diameter variation was generally at least 40% and typically of the order of 50-90%.

**Preparation of the mixtures of the components of the various layers, of the core and of the coating of the GRT compacts or tablets.**

The mixing of the components, each optionally sieved, was carried out in amber-colored, cylindrical glass containers with screw cap equipped with a Teflon stopper, or in suitable stainless steel containers and was performed in a Turbula® mixer (3D blender mixer WAB Group) until the mixture of the components was completely uniform, generally in the following manner:

1. a homogeneous core was formed composed of the minority component(s) and of an identical quantity by weight of active substance

2. the whole remaining active substance was added

3. the majority excipients were added in a weight amount identical to the weight amount of the powder contained in the container

4. the whole remaining amount of the majority excipients was added.

For all the mixtures, each aliquot of powder was mixed for a time which depended on the masses involved, in general for the largest quantities up to a maximum of 30-40 minutes.

**Preparation of compacts or cylindrical tablets**

The cylindrical single-layer compacts, having a 13-mm diameter, were obtained by applying the different selected compression forces on a weight of the premixed powder corresponding to about 300 mg, using an oil hydraulic press of the Carver type equipped for measuring the applied force or, alternatively, a rotary tableting press Ronchi with 8 punches equipped for measuring compression forces.

For bilayer compacts, a layer of the weight of about 200 mg was precompacted at about 7 kN; then, about 200 mg of the second mixture were added into the matrix and a 25-kN compression force was applied.

For the tri-layer disks, about 136 mg of the mixture of the intermediate layer were precompacted at 7 kN; then, about 136 mg of the layer containing sodium bicarbonate were added to said layer and compressed at about 16 kN; finally, 133 mg of the layer containing the super-disintegrating agent were added and a 25-kN compression force was applied.

## Preparation of the core-tablets (e.g. containing metformin hydrochloride)

The coated tablets of the core-tablet type were obtained by coating the core using a Carver press. For many compositions, a cylindrical compact with a 150-mg weight was thus obtained by applying a given compression force. The core was then coated in a 13-mm matrix. Thus, tablets of about 450 mg were obtained by applying the selected compression force.

The compression of the cores contained in the coated tablets (e.g. ME8-ME9) was performed using a rotary tableting press Ronchi equipped for measuring compression forces using flat-shaped punches of 7-mm diameter.

The cores obtained by applying a given compression force (for example 32 kN to 93 $\pm$ 0.5 mg of powder in the case of tablets ME8 and ME9) had a thickness of 1.95 mm. Instead, the final tablets of 10-mm or 13-mm diameter, obtained by centering the core between the two coating layers in an oil hydraulic press of the Carver type, were produced by applying a compression force of 5 Tons for one minute to a total quantity of powder of 270 mg for the formulation ME8; for the tablets ME9, a compression force of 3 Tons was applied for one minute, thereby obtaining tablets with a 13-mm diameter and a final weight of about 900 mg.

Instead, the cores of formulations ME10 and ME11 were obtained by compressing at 5 tons (with Carver press) respectively 100 and 150 mg of premixed powder. The cores obtained (10-mm diameter) were centered between two coating layers and compressed at 3 tons for 1 minute by Carver press. The final compacts had a 13-mm diameter and a 900-mg (ME10) and a 950-mg weight (ME11).

## HEAT TREATMENTS

They were performed at temperature Tx (range 80° - 250°C) for time tx (range 1-60 min) in ventilated oven. Each compact (at most 3) was placed inside a metal basket situated inside the oven and treated according to the following steps:

1. Compact conditioning at 30°C for 0.1 min

2. Heating to Tx (e.g. 150°C) with heating gradient of 30°C/min

3. Treatment for the selected time tx (e.g. 5, 15 or 30 min)

4. Cooling in the oven to 30°C

5. Compact conditioning at 30°C for 5 min before taking the compact

The thermally treated compacts were weighed and measured to determine the weight loss, the variation of the axial thickness and of the diameter.

DETERMINING THE EROSION DEGREE OF THE MATRIX (EROSION INDEX - EI) AFTER DISSOLUTION TEST OR PERMANENCE IN FLUIDS SIMULATING THE GASTRIC FLUID

For evaluating the erodibility of the compacts, at the end of the dissolution test the residual compact was taken, let drip on a plastic gauze for 30 s and weighed, it was then placed in a glass capsule and let dry at room temperature for at least 48 hours, and in any case for a time sufficient to reach a constant final weight.

The Erosion Index (EI) was calculated as follows:

$$\mathrm{EI}\ \% = (\mathrm{w_{cT}} - \mathrm{w_{re}})\ /\ \mathrm{w_{cT}} * 100$$

wherein

$w_{cT}$ = initial weight of the compact (corresponding to the weight after heat treatment for the thermally treated compacts)

$w_{re}$ = residual weight of the compact taken at the end of the dissolution test after drying

In the evaluation of the erosion index, one should always consider the weight loss due to the release of the drug and of other soluble components still present in the composition of the matrix after the heat treatment.

DETERMINING THE SWELLING DEGREE (SWELLING INDEX - SI)

This study was performed on compacts recovered at determined times during simulated dissolution tests (performed with the only purpose of evaluating SI variations, therefore not measuring the release of the active ingredient at the various time points) or at the end of the dissolution test.

Each compact was taken from the vessel of the dissolution test with a spatula, gently dried with absorbent paper and weighed.

The Swelling Index (SI) was calculated as follows:

$$SI \% = (W_f - W_{cT})/W_{cT} *100$$

wherein

$W_f$ = weight of the compact recovered at the defined time point or at the end of the dissolution test

$W_{ct}$ = initial weight of the compact (corresponding to the weight after heat treatment for the thermally treated compacts).

DISSOLUTION TEST

The dissolution test was performed in a Distek Premiere 5100 apparatus. The dissolution medium (1 L), contained in a glass vessel, was thermostated to 37 $\pm$ 0.1 °C and the rotation speed of the paddles was fixed at 50 rpm.

The determination of the dissolved active ingredient was carried out through DAD UV-VIS Agilent Technologies 8453, automated with peristaltic pump and tube-carrier system "Multicell Transport for Agilent 8453", controlled by the relevant software. The sampling time was set to follow dissolution even up to 24 hours.

The analysis was performed at an analytical wavelength set according to the spectrum of the active ingredient as shown in Table 38.

*Table 38*

| Active ingredient | Analytical wavelength (nm) |
|---|---|
| Diltiazem hydrochloride | 236 |
| Metformin hydrochloride | 233 |
| Baclofen | 220 |
| Levodopa | 210 |

The dissolution test at pH 1.2 was carried out in a solution of 0.1 N HCl, whereas that with pH change was carried out with the following procedure:

I step - Dissolution in acidic environment with 760 mL of 0.1 N HCl per vessel;

II step - Dissolution at pH 6.8: 240 mL phosphate buffer (76.026 g of $Na_3PO_4 \cdot 12 H_2O$ per $H_2O$ liter) were added to each vessel after 2 hours from test start, as from USP monograph, or after 4 hours from test start to better adapt to the type of GRT study.

The determination was performed versus a standard of concentration equal to the concentration which was determined when 100% of the content of the tablet was released in the dissolution medium.

**Claims**

1. A method for the preparation of a compact bioadhesive, gastroretentive matrix, which includes the following steps:

- preparing a homogeneous mixture of powders comprising at least one alkylcellulose, a water-insoluble, water-swellable cross-linked polycarboxylic polymer and at least one pH regulator selected from the group consisting of carbonate and bicarbonate of an alkali metal or an alkaline earth metal, and hydrogen phosphate of an alkali metal;
- preparing compressed or compact units starting from said mixture of powders by direct compression or dry compaction;
- subjecting the compressed or compact units thus obtained to heating at a temperature between 80°C and 250°C, preferably between 100°C and 160°C, for a time between 1-60 minutes.

2. The method according to claim 1, for the preparation of bioadhesive, gastroretentive compressed units, preferably pharmaceutical tablets, containing at least one active substance, preferably a pharmacologically active substance, and suitable for a prolonged or controlled release of said at least one active substance, wherein said homogeneous mixture of powders also comprises at least one active substance.

3. The method according to any one of claims 1-2, wherein said alkylcellulose is selected from the group consisting of methylcellulose and ethylcellulose, preferably ethylcellulose.

4. The method according to any one of claims 1-3, wherein said water-insoluble, water-swellable cross-linked polycarboxylic polymer is selected from carbopol and Polycarbophil and is preferably Polycarbophil.

5. The method according to any one of claims 1-4, wherein said pH regulator is selected from the group consisting of carbonate and bicarbonate of Li, Na, K, Ca and Mg and hydrogen phosphate of Li, Na and K.

6. The method according to claim 2, wherein said homogeneous mixture of powders comprises at least one active substance, ethylcellulose, Polycarbophil and a UGE016WEP pH regulator selected from the group consisting of carbonate and bicarbonate of Li, Na, K, Ca and Mg and hydrogen phosphate of Li, Na and K.

7. The method according to claim 6, wherein Polycarbophil constitutes 15-75%, preferably 30-65%, by weight, of the total weight of said mixture of powders, the pH regulator constitutes 10-51%, preferably 15-45%, by weight, of the total weight of said mixture of powders and the pH regulator and Polycarbophil are present in said mixture of powders in a weight ratio ranging from 1:3 to 1:1.

8. The method according to claim 7, wherein ethylcellulose constitutes 0.1-80%, preferably 10-20%, by weight, of the total weight of said mixture of powders and wherein ethylcellulose and Polycarbophil are present in said mixture of powders in a weight ratio ranging from 1:10 to 4:1.

9. The method according to any one of claims 6-8, wherein said at least one active substance is contained in said mixture of powders in an amount ranging from 0.001 ppm to 50% by weight of the total weight of the mixture.

10. A bioadhesive and gastroretentive prolonged-release tablet, preferably of the multilayer or core-tablet type, comprising at least one active substance, at least one alkylcellulose, a water-insoluble, water-swellable cross-linked polycarboxylic polymer, preferably consisting of Polycarbophil, and at least one pH regulator selected from the group consisting of carbonate and bicarbonate of an alkali metal or an alkaline earth metal and hydrogen phosphate of an alkali metal, said tablet having a floating lag time of less than 60 seconds, preferably less than or equal to 20 seconds, more preferably less than or equal to 10 seconds, measured in 0.1 M HCl (1 L), pH 1.2, at 37°C, wherein said tablet is obtainable with the method according to claim 2 or according to any one of claims 3 to 9, when dependent on claim 2.

11. The bioadhesive and gastroretentive tablet according to claim 10, wherein said alkylcellulose is selected from the group consisting of methylcellulose and ethylcellulose, preferably ethylcellulose.

12. The bioadhesive and gastroretentive tablet according to claim 10 or 11, having a total floating time of at least 4 hours, preferably of at least 8 hours, measured in 0.1 M HCl (1 L), pH 1.2, at 37°C.

13. The bioadhesive and gastroretentive tablet according to any one of claims 10-12, having a percentage swelling index (SI- swelling index %) between 180% and UGE016WEP 800%, preferably between 350% and 700%, assessed at the end of a dissolution test performed in 0.1 M HCl (1 L), pH 1.2, at 37°C, and having an erosion index (EI) in the range of 0.1%-75%, preferably 7%-40%, assessed at the end of a dissolution test performed in 0.1 M HCl (1 L), pH 1.2, at 37°C.

14. The bioadhesive and gastroretentive tablet according to any one of claims 10-13, **characterized in that** it has a controlled release and a release kinetics of the active substance of substantially zero order in an aqueous solution at pH values between 1 and 8.

15. The tablet according to any one of claims 10-14, wherein said at least one alkylcellulose is ethylcellulose, said water-insoluble, water-swellable cross-linked polycarboxylic polymer is Polycarbophil, and said at least one pH regulator is selected from the group consisting of carbonate and bicarbonate of Li, Na, K, Ca and Mg and hydrogen phosphate of Li, Na and K.

16. The tablet according to claim 15, wherein Polycarbophil constitutes 15-75%, preferably 30-65%, by weight, of the total weight of said tablet, the pH regulator constitutes 10-51%, preferably 15-45%, by weight, of the total weight of said tablet and the pH regulator and Polycarbophil are present in a weight ratio ranging from 1:3 to 1:1.

17. The tablet according to claim 16, wherein ethylcellulose constitutes 0.1-80%, preferably 10-20%, by weight, of the total weight of said tablet and wherein ethylcellulose and Polycarbophil are present in a weight ratio ranging from 1:10 to 4:1.

18. The tablet according to any one of claims 15-17, wherein said at least one active substance is contained in said tablet in an amount ranging from 0.001 ppm to 50% by weight of the total weight of the tablet and is preferably selected from the group consisting of baclofen, diltiazem, levodopa, metformin, and pharmaceutically acceptable salts thereof.

19. The tablet according to any one of claims 10-18, **characterized in that** it is a tablet of the core-tablet type, which has a weight ratio between core and coating of between 1:1.5 and 1:3, preferably between 1:1.8 and 1:2.2.


**Patentansprüche**

1. Verfahren zur Herstellung einer kompakten bioadhäsiven, gastroretentiven Matrix, umfassend die folgenden Schritte:

   - Herstellen eines homogenen Pulvergemisches, das mindestens eine Alkylcellulose, ein wasserunlösliches, wasserquellbares, vernetztes Polycarbonsäurepolymer sowie mindestens einen pH-Regulator ausgewählt aus der Gruppe bestehend aus Carbonat und Bicarbonat eines Alkalimetalls oder Erdalkalimetalls und Hydrogenphosphat eines Alkalimetalls umfasst;
   - Herstellen von gepressten oder kompakten Einheiten aus dem genannten Pulvergemisch durch Direktverpressung oder Trockenkompaktierung;
   - Erhitzen der so erhaltenen gepressten oder kompakten Einheiten bei einer Temperatur zwischen 80 °C und 250 °C, vorzugsweise zwischen 100 °C und 160 °C, für eine Dauer zwischen 1 und 60 Minuten.

2. Verfahren nach Anspruch 1 zur Herstellung bioadhäsiver, gastroretentiver gepresster Einheiten, vorzugsweise pharmazeutischer Tabletten, die mindestens einen Wirkstoff, vorzugsweise einen pharmakologisch aktiven Wirkstoff, enthalten und geeignet sind für eine verlängerte oder gesteuerte Freisetzung des genannten mindestens einen Wirkstoffs, wobei das genannte homogene Pulvergemisch ferner mindestens einen Wirkstoff umfasst.

3. Verfahren nach einem der Ansprüche 1 oder 2, wobei die genannte Alkylcellulose ausgewählt ist aus der Gruppe bestehend aus Methylcellulose und Ethylcellulose, vorzugsweise Ethylcellulose.

4. Verfahren nach einem der Ansprüche 1 bis 3, wobei das genannte wasserunlösliche, wasserquellbare, vernetzte Polycarbonsäurepolymer ausgewählt ist aus Carbopol und Polycarbophil und vorzugsweise Polycarbophil ist.

5. Verfahren nach einem der Ansprüche 1 bis 4, wobei der genannte pH-Regulator ausgewählt ist aus der Gruppe bestehend aus Carbonat und Bicarbonat von Li, Na, K, Ca und Mg sowie Hydrogenphosphat von Li, Na und K.

6. Verfahren nach Anspruch 2, wobei das genannte homogene Pulvergemisch mindestens einen Wirkstoff, Ethylcellulose, Polycarbophil und einen pH-Regulator ausgewählt aus der Gruppe bestehend aus Carbonat und Bicarbonat von Li, Na, K, Ca und Mg sowie Hydrogenphosphat von Li, Na und K umfasst.

7. Verfahren nach Anspruch 6, wobei Polycarbophil 15-75 Gew.-%, vorzugsweise 30-65 Gew.-%, des Gesamtgewichts des genannten Pulvergemisches ausmacht, der pH-Regulator 10-51 Gew.-%, vorzugsweise 15-45 Gew.-%, des Gesamtgewichts des genannten Pulvergemisches ausmacht und der pH-Regulator und Polycarbophil in dem genannten Pulvergemisch in einem Gewichtsverhältnis von 1:3 bis 1:1 vorliegen.

8. Verfahren nach Anspruch 7, wobei Ethylcellulose 0,1-80 Gew.-%, vorzugsweise 10-20 Gew.-%, des Gesamtgewichts des genannten Pulvergemisches ausmacht und wobei Ethylcellulose und Polycarbophil in dem genannten Pulvergemisch in einem Gewichtsverhältnis von 1:10 bis 4:1 vorliegen.

9. Verfahren nach einem der Ansprüche 6 bis 8, wobei der genannte mindestens eine Wirkstoff in dem genannten Pulvergemisch in einer Menge von 0,001 ppm bis 50 Gew.-% des Gesamtgewichts des Gemisches enthalten ist.

10. Bioadhäsive und gastroretentive Retardtablette, vorzugsweise vom Mehrschicht- oder Kern-Tablettentyp, umfassend mindestens einen Wirkstoff, mindestens eine Alkylcellulose, ein wasserunlösliches, wasserquellbares, vernetztes Polycarbonsäurepolymer, vorzugsweise bestehend aus Polycarbophil, sowie mindestens einen pH-Regulator ausgewählt aus der Gruppe bestehend aus Carbonat und Bicarbonat eines Alkalimetalls oder Erdalkalimetalls und Hydrogenphosphat eines Alkalimetalls, wobei die Tablette eine Aufschwimmverzögerungszeit von weniger als 60 Sekunden, vorzugsweise kleiner oder gleich 20 Sekunden, besonders bevorzugt kleiner oder gleich 10 Sekunden, aufweist, gemessen in 0,1 M HCl (1 L), pH 1,2, bei 37 °C, und wobei die Tablette nach dem Verfahren gemäß Anspruch 2 oder gemäß einem der Ansprüche 3 bis 9, soweit von Anspruch 2 abhängig, erhältlich ist.

11. Bioadhäsive und gastroretentive Tablette nach Anspruch 10, wobei die genannte Alkylcellulose ausgewählt ist aus der Gruppe bestehend aus Methylcellulose und Ethylcellulose, vorzugsweise Ethylcellulose.

12. Bioadhäsive und gastroretentive Tablette nach Anspruch 10 oder 11, mit einer gesamten Aufschwimmzeit von mindestens 4 Stunden, vorzugsweise mindestens 8 Stunden, gemessen in 0,1 M HCl (1 L), pH 1,2, bei 37 °C.

13. Bioadhäsive und gastroretentive Tablette nach einem der Ansprüche 10 bis 12, mit einem prozentualen Quellindex (SI - swelling index %) zwischen 180 % und 800 %, vorzugsweise zwischen 350 % und 700 %, bestimmt am Ende eines Auflösungstests in 0,1 M HCl (1 L), pH 1,2, bei 37 °C, und mit einem Erosionsindex (EI) im Bereich von 0,1 % bis 75 %, vorzugsweise 7 % bis 40 %, bestimmt am Ende eines Auflösungstests in 0,1 M HCl (1 L), pH 1,2, bei 37 °C.

14. Bioadhäsive und gastroretentive Tablette nach einem der Ansprüche 10 bis 13, **dadurch gekennzeichnet, dass** sie eine gesteuerte Freisetzung und eine Freisetzungskinetik des Wirkstoffs im Wesentlichen nullter Ordnung in einer wässrigen Lösung bei pH-Werten zwischen 1 und 8 aufweist.

15. Tablette nach einem der Ansprüche 10 bis 14, wobei die genannte mindestens eine Alkylcellulose Ethylcellulose ist, das genannte wasserunlösliche, wasserquellbare, vernetzte Polycarbonsäurepolymer Polycarbophil ist und der genannte mindestens eine pH-Regulator ausgewählt ist aus der Gruppe bestehend aus Carbonat und Bicarbonat von Li, Na, K, Ca und Mg sowie Hydrogenphosphat von Li, Na und K.

16. Tablette nach Anspruch 15, wobei Polycarbophil 15-75 Gew.-%, vorzugsweise 30-65 Gew.-%, des Gesamtgewichts der genannten Tablette ausmacht, der pH-Regulator 10-51 Gew.-%, vorzugsweise 15-45 Gew.-%, des Gesamtgewichts der genannten Tablette ausmacht und der pH-Regulator und Polycarbophil in einem Gewichtsverhältnis von 1:3 bis 1:1 vorliegen.

17. Tablette nach Anspruch 16, wobei Ethylcellulose 0,1-80 Gew.-%, vorzugsweise 10-20 Gew.-%, des Gesamtgewichts der genannten Tablette ausmacht und wobei Ethylcellulose und Polycarbophil in einem Gewichtsverhältnis von 1:10 bis 4:1 vorliegen.

18. Tablette nach einem der Ansprüche 15 bis 17, wobei der genannte mindestens eine Wirkstoff in der genannten Tablette in einer Menge von 0,001 ppm bis 50 Gew.-% des Gesamtgewichts der Tablette enthalten ist und vorzugsweise ausgewählt ist aus der Gruppe bestehend aus Baclofen, Diltiazem, Levodopa, Metformin und pharmazeutisch verträglichen Salzen davon.

19. Tablette nach einem der Ansprüche 10 bis 18, **dadurch gekennzeichnet, dass** es sich um eine Tablette vom Kern-Tablettentyp handelt, die ein Gewichtsverhältnis zwischen Kern und Überzug von 1:1,5 bis 1:3, vorzugsweise von 1:1,8 bis 1:2,2 aufweist.

**Revendications**

1. Procédé de préparation d'une matrice bioadhésive compacte, gastrorétentive, comprenant les étapes suivantes:

   - préparation d'un mélange homogène de poudres comprenant au moins une alkylcellulose, un polymère polycarboxylique réticulé insoluble dans l'eau et gonflable dans l'eau, et au moins un régulateur de pH choisi dans le groupe constitué par le carbonate et le bicarbonate d'un métal alcalin ou d'un métal alcalino-terreux, et l'hydrogénophosphate d'un métal alcalin;
   - préparation d'unités comprimées ou compactes à partir dudit mélange de poudres par compression directe ou compactage à sec;
   - soumettre les unités comprimées ou compactes ainsi obtenues à un chauffage à une température comprise entre 80 °C et 250 °C, de préférence entre 100 °C et 160 °C, pendant une durée comprise entre 1 et 60 minutes.

2. Procédé selon la revendication 1, pour la préparation d'unités comprimées bioadhésives et gastrorétentives, de préférence des comprimés pharmaceutiques, contenant au moins une substance active, de préférence une substance pharmacologiquement active, et adaptées à une libération prolongée ou contrôlée de ladite au moins une substance active, dans lequel ledit mélange homogène de poudres comprend également au moins une substance active.

3. Procédé selon l'une quelconque des revendications 1 à 2, dans lequel ladite alkylcellulose est choisie dans le groupe constitué par la méthylcellulose et l'éthylcellulose, de préférence l'éthylcellulose.

4. Procédé selon l'une quelconque des revendications 1 à 3, dans lequel ledit polymère polycarboxylique réticulé, insoluble dans l'eau et gonflable dans l'eau, est choisi parmi le carbopol et le Polycarbophil et est de préférence le Polycarbophil.

5. Procédé selon l'une quelconque des revendications 1 à 4, dans lequel ledit régulateur de pH est choisi dans le groupe constitué par le carbonate et le bicarbonate de Li, Na, K, Ca et Mg et l'hydrogénophosphate de Li, Na et K.

6. Procédé selon la revendication 2, dans lequel ledit mélange homogène de poudres comprend au moins une substance active, de l'éthylcellulose, du Polycarbophil et un régulateur de pH choisi dans le groupe constitué par le carbonate et le bicarbonate de Li, Na, K, Ca et Mg et l'hydrogénophosphate de Li, Na et K.

7. Procédé selon la revendication 6, dans lequel le Polycarbophil constitue 15 à 75 %, de préférence 30 à 65 %, en poids, du poids total dudit mélange de poudres, le régulateur de pH constitue 10 à 51 %, de préférence 15 à 45 %, en poids, du poids total dudit mélange de poudres, et le régulateur de pH et le Polycarbophil sont présents dans ledit mélange de poudres dans un rapport pondéral compris entre 1:3 et 1:1.

8. Procédé selon la revendication 7, dans lequel l'éthylcellulose constitue 0,1 à 80 %, de préférence 10 à 20 %, en poids, du poids total dudit mélange de poudres et dans lequel l'éthylcellulose et le Polycarbophil sont présents dans ledit mélange de poudres dans un rapport pondéral compris entre 1:10 et 4:1.

9. Procédé selon l'une quelconque des revendications 6 à 8, dans lequel ladite au moins une substance active est contenue dans ledit mélange de poudres dans une quantité comprise entre 0,001 ppm et 50 % en poids du poids total du mélange.

10. Comprimé bioadhésif et gastrorétentif à libération prolongée, de préférence du type multicouche ou à noyau, comprenant au moins une substance active, au moins une alkylcellulose, un polymère polycarboxylique réticulé insoluble dans l'eau et gonflable dans l'eau, de préférence constitué par le Polycarbophil, et au moins un régulateur de pH choisi dans le groupe constitué par le carbonate et le bicarbonate d'un métal alcalin ou d'un métal alcalino-terreux et l'hydrogénophosphate d'un métal alcalin, ledit comprimé ayant un temps de latence de flottaison inférieur à 60 secondes, de préférence inférieur ou égal à 20 secondes, plus préférablement inférieur ou égal à 10 secondes, mesuré dans 0,1 M HCl (1 L), pH 1,2, à 37 °C, dans lequel ledit comprimé peut être obtenu par le procédé selon la revendication 2 ou selon l'une quelconque des revendications 3 à 9, lorsqu'il dépend de la revendication 2.

11. Comprimé bioadhésif et gastrorétentif selon la revendication 10, dans lequel ladite alkylcellulose est choisie dans le groupe constitué par la méthylcellulose et l'éthylcellulose, de préférence l'éthylcellulose.

**12.** Comprimé bioadhésif et gastrorétentif selon la revendication 10 ou 11, ayant un temps de flottaison total d'au moins 4 heures, de préférence d'au moins 8 heures, mesuré dans 0,1 M HCl (1 L), pH 1,2, à 37 °C.

**13.** Comprimé bioadhésif et gastrorétentif selon l'une quelconque des revendications 10 à 12, ayant un indice de gonflement en pourcentage (SI- indice de gonflement %) compris entre 180 % et 800 %, de préférence entre 350 % et 700 %, évalué à la fin d'un test de dissolution effectué dans 0,1 M HCl (1 L), pH 1,2, à 37 °C, et ayant un indice d'érosion (EI) compris entre 0,1 % et 75 %, de préférence entre 7 % et 40 %, évalué à la fin d'un test de dissolution réalisé dans 0,1 M HCl (1 L), pH 1,2, à 37 °C.

**14.** Comprimé bioadhésif et gastrorétentif selon l'une quelconque des revendications 10 à 13, **caractérisé en ce qu'**il présente une libération contrôlée et une cinétique de libération de la substance active d'ordre pratiquement zéro dans une solution aqueuse à des valeurs de pH comprises entre 1 et 8.

**15.** Comprimé selon l'une quelconque des revendications 10 à 14, dans lequel ladite au moins une alkylcellulose est l'éthylcellulose, ledit polymère polycarboxylique réticulé insoluble dans l'eau et gonflable dans l'eau est le Polycarbophil, et ledit au moins un régulateur de pH est choisi dans le groupe constitué par le carbonate et le bicarbonate de Li, Na, K, Ca et Mg et l'hydrogénophosphate de Li, Na et K.

**16.** Comprimé selon la revendication 15, dans lequel le Polycarbophil constitue 15 à 75 %, de préférence 30 à 65 %, en poids, du poids total dudit comprimé, le régulateur de pH représente 10 à 51 %, de préférence 15 à 45 %, en poids, du poids total dudit comprimé, et le régulateur de pH et le Polycarbophil sont présents dans un rapport pondéral compris entre 1:3 et 1: 1.

**17.** Comprimé selon la revendication 16, dans lequel l'éthylcellulose constitue 0,1 à 80 %, de préférence 10 à 20 %, en poids, du poids total dudit comprimé et dans lequel l'éthylcellulose et le Polycarbophil sont présents dans un rapport pondéral compris entre 1:10 et 4:1.

**18.** Comprimé selon l'une quelconque des revendications 15 à 17, dans lequel ladite au moins une substance active est contenue dans ledit comprimé en une quantité comprise entre 0,001 ppm et 50 % en poids du poids total du comprimé et est de préférence choisie dans le groupe constitué par le baclofène, le diltiazem, la lévodopa, la metformine et leurs sels pharmaceutiquement acceptables.

**19.** Comprimé selon l'une quelconque des revendications 10 à 18, **caractérisé en ce qu'**il s'agit d'un comprimé de type comprimé à noyau, dont le rapport pondéral entre le noyau et l'enrobage est compris entre 1:1,5 et 1:3, de préférence entre 1:1,8 et 1:2,2.

Fig. 1 DSC and TGA profiles of sodium carbonate

Fig. 2 DSC and TGA profiles of sodium bicarbonate

**Comparison X9A305 and X9A306 T and NT at 15 kN and 25 kN (Na$_2$CO$_3$)**

Fig. 3 Comparison of release profiles of DTZ (average ± standard deviation) in 0.1 N HCl, pH 1.2, from tablets X9A305 (15kN) and X9A306 (25 kN) NT (n=3) and T (n=3).

Fig. 4 Average swelling profiles (average ± standard deviation) of two tablets containing Na$_2$CO$_3$ obtained with a different compression force (15 X9A305 and 25 kN X9A306) and thermally treated at 150°C*15 min.

**Comparison X9A305 T and NT at 15 kN (Na$_2$CO$_3$)**

Fig. 5 Comparison of release profiles of DTZ (average ± standard deviation) in 0.1 N HCl, pH 1.2, from tablets X9A305 15kN NT (n=3) and T at two heat treatment conditions (n=3). For each curve, the corresponding EI value (average±SD), obtained from the matrices recovered at the end of the dissolution test, is shown.

**Comparison X9A306 at different T and NT at 25kN (Na$_2$CO$_3$)**

Fig. 6 Comparison of release profiles of DTZ (average ± standard deviation) in 0.1 N HCl, pH 1.2, from tablets X9A306 NT (n=3) and T at various heat treatment conditions (n=3). For each curve, the corresponding EI value (average±SD), obtained from the matrices recovered at the end of the dissolution test, is shown.

Fig. 7 Comparison of release profiles of DTZ (average ± standard deviation) in 0.1 N HCl, pH 1.2, from tablets X9A307 NT (n=3) and T at various conditions (n=3). For each curve, the corresponding EI value (average±SD), obtained from the matrices recovered at the end of the dissolution test, is shown.

Fig. 8 Comparison of swelling profiles (average swelling index ± SD) in 0.1 N HCl, pH 1.2, of tablets X9A306 (containing carbonate) and X9A307 (containing bicarbonate) treated at 150°C x 15 min.

**Comparison X9A307 T and NT at 25 kN (NaHCO₃)**

Fig. 9 Comparison of release profiles of DTZ (average ± standard deviation) with pH change from tablets X9A307 NT (n=3) and T (n=3); after the first two hours at pH 1.2, the pH was brought to 6.8. For each curve, the corresponding EI value (average±SD), obtained from the matrices recovered at the end of the dissolution test, is shown.

Fig. 10 Compacts X9A307 T 150°C x 15 min recovered at the end of the dissolution test of Fig. 9, carried out with pH change.

**Comparison W9A018 (NA$_2$HPO$_4$) with X9A306 (Na$_2$CO$_3$) and X9A307 (NaHCO$_3$) T 150*15**

EI%=46.44±2.31

EI%=37.08±0.35

EI%=40.37±13.14

—▲— average W9A018 T 150*15

—※— average X9A306 T 150*15

········ average X9A307 T 150*15

%Drug released

Time (min)

Fig. 11 Comparison of release profiles of DTZ (average ± standard deviation) in 0.1 N HCl, pH 1.2, from tablets W9A018 (n=2) with tablets X9A306 (n=3) and X9A307 (n=3), T 150°Cx15. For each curve, the corresponding EI value (average±SD), obtained from the matrices recovered at the end of the dissolution test, is shown.

Fig. 12 Tablets W9A018 T 150°C x 15 min recovered at the end of the dissolution test with pH change.

**Comparison X9A308 at different T and NT at 25 kN (Na₂CO₃)**

Fig. 13 Comparison of release profiles of baclofen (average ± standard deviation) in 0.1 N HCl, pH 1.2, from compacts X9A308 25 kN NT (n=3) and subjected to different heat treatments: 150°C x 15 min (n=3), 140°C x 5 min (n=3), 130°C x 15 min (n=3).

For each curve, the corresponding EI value (average±SD), obtained from the matrices recovered at the end of the dissolution test, is shown.

**Comparison X9A309 T and NT at 25 kN (NaHCO₃)**

Fig. 14 Comparison of release profiles of baclofen (average ± standard deviation) in 0.1 N HCl, pH 1.2, from compacts X9A309 25 kN NT (n=3) and T (n=3). For each curve, the corresponding EI value (average±SD), obtained from the matrices recovered at the end of the dissolution test, is shown.

Fig. 15 Comparison of release profiles of DTZ (expressed in mg, average ± standard deviation) in 0.1 N HCl, pH 1.2, from compacts W9A017 (n=2) with W9A015 (n=3) and W9A016 (n=3), T 150x30.

Fig. 16 Comparison of release profiles of MH (average ± standard deviation) in 0.1 N HCl, pH 1.2, from tablets ME8 T 150°C x 15 min and NT (n=3).

Fig. 17 Comparison of release profiles of MH (average ± standard deviation) in 0.1 N HCl, pH 1.2, from tablets ME8 T 150°C x 15 min, ME9 T 150°C x 15 min and of ME8 + ME9 150°C x 15 min.

Fig. 18 Comparison of release profiles of MH (average ± standard deviation) of tablets ME8, ME9 and of the co-administration ME8 + ME9 in 0.1 N HCl, pH 1.2 for the first 4 h and at pH 6.8 until the end of the test.

Fig. 19 Comparison of release profiles of MH (expressed in mg, average ± standard deviation) in 0.1 N HCl, pH 1.2, from tablets ME8 + ME9 T 150°C x 15 min, ME8 + ME10 T 150°C x 15 min and of ME8 + ME11 T 150°C x 15 min.

Fig. 20 Comparison of the dissolution profiles of baclofen (average ± standard deviation) in 0.1 N HCl, pH 1.2 from *core-tablets* T and NT of the composition described in Table 22.

2

Fig. 21 Swelling profile (SI, average ± SD) of tablets of the dissolution test of Fig. 20 in 0.1 N HCl, pH 1.2.

Fig. 22 Profile of variation of the thickness (average ± SD) of tablets of the dissolution test of Fig. 20 in 0.1 N HCl, pH 1.2

Fig. 23 Profile of variation of the diameter (average ± SD) of tablets of the dissolution test of Fig. 20 in 0.1 N HCl, pH 1.2

Fig. 24 Comparison of release profiles of baclofen (average ± standard deviation) in 0.1 N HCl, pH 1.2 of *core-tablets* with the same percentage composition of the tablets of Fig. 20 but different diameter (10 mm) and weight (core: about 90 mg, total: about 280 mg).

Fig. 25 Comparison of release profiles of baclofen (average ± standard deviation) in 0.1 N HCl, pH 1.2 from *core-tablets* having the same composition but subjected to different heat treatment times (15 and 25min)

Fig. 26 Comparison of release profiles of baclofen (average ± standard deviation) of *core-tablets* T and NT in 0.1 N HCl, pH 1.2 for the first 4 h and at pH 6.8 until the end of the test. The vertical line indicates the time at which the pH change was performed.

Fig. 27 Tablets CRB250314 T 150°C x 15 min recovered at the end of the dissolution test (DT) with pH change. The diameter measured after the DT was 46.2 mm.

Fig. 28 Tablet according to the invention after 4 hours of treatment in the DGM apparatus in the state simulating the "on an empty stomach" condition.

Fig. 29 Tablet according to the invention after 6 hours of treatment in the DGM apparatus in the state simulating the "on a full stomach" condition.

Fig. 30 Release profile of DTZ (average ± standard deviation) in 0.1 N HCl, pH 1.2, from tablets coated according to the invention (example 13) compared with tablets of identical composition but not subjected to heat treatment.

Fig. 31 Release profile of LD5a containing Levodopa (average ± standard deviation) in 0.1 N HCl, pH 1.2, from coated tablets according to the invention (example 14) compared with tablets of identical composition but not subjected to heat treatment.

Fig. 32: Profile of variation of the diameter of tablets CRB110314 according to the invention at predetermined time points (Example 15).

Fig. 33: values of the swelling index SI% of tablets CRB110314 at predetermined time points (Example 15).

Fig. 34: Profile of variation of the diameter of tablets F14M1 lacking active substance at predetermined time points (Example 16).

Fig. 35: values of the swelling index SI% of tablets F14M1 lacking active substance at predetermined time points (Example 16).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019069108 A, Fenyvesi
- WO 2020006278 A, Shah
- US 3901232 A, Michaels
- WO 2018232413 A, Sandhu
- CN 103585098

- EP 2105133 A
- WO 03097018 A
- EP 2344140 B1
- EP 1907108 A

### Non-patent literature cited in the description

- **CHEN et al.** Swelling/Floating Capability and Drug Release Characterizations of Gastroretentive Drug Delivery System Based on a Combination of Hydroxyethyl Cellulose and Sodium Carboxymethyl Cellulose. *PLoS ONE*, 2015, vol. 10 (1), e0116914
- **KIM et al.** Preparation and evaluation of non-effervescent gastroretentive tablets containing pregabalin for once-daily administration and dose proportional pharmacokinetics. *Int J Pharm*, 2018, vol. 550 (1-2), 160-169
- **WEI et al.** Gastro-floating bilayer tablets for the sustained release of metformin and immediate release of pioglitazone: Preparation and in vitro/in vivo evaluation. *Int J Pharm*, 2014, vol. 476 (1-2), 223-231
- **BAUMGARTNER et al.** Optimisation of floating matrix tablets and evaluation of their gastric residence time. *Int J Pharm*, 2000, vol. 195, 125-135
- **JIMÉNEZ-MARTINEZ et al.** Sustained delivery of captopril from floating matrix tablets.. *Int J Pharm*, 2008, vol. 362 (1-2), 37-43
- **KISHAN et al.** Drug-excipient interaction during formulation development in vitro and in vivo evaluation of gastroretentive drug delivery system for nizatidine. *Int J Pharm Sci Nanotech*, 2013, vol. 6 (4)
- **JIMÉNEZ-MARTINEZ et al.** Sustained delivery of captopril from floating matrix tablets. *Int J Pharm*, 2008, vol. 362 (1-2), 37-43
- **FUKUDA et al.** Floating hot-melt extruded tablets for gastroretentive controlled drug release system. *J Control Release*, 2006, vol. 115 (2), 121-9
- **MEKA et al.** Design and evaluation of polymeric coated minitablets as multiple unit gastroretentive floating drug delivery systems for furosemide. *J Pharm Sci*, 2009, vol. 98 (6), 2122-32
- **HAMPSON et al.** Alginate-antiacid combinations: raft formation and gastric retention studies. *Drug Dev Ind Pharm*, 2010, vol. 36 (5), 614-23

- **OMIDIAN et al.** Advances in superporous hydrogels. *J Control Release*, 2005, vol. 102 (1), 3-12
- **EL-SAID et al.** Baclofen novel gastroretentive extended release gellan gum superporous hydrogel hybrid system: in vitro and in vivo evaluation. *Drug Deliv*, 2016, vol. 23 (1), 101-12
- **PATIL et al.** Recent advancements in mucoadhesive floating drug delivery systems: A mini-review. *J Drug Deliv Sci Tech*, 2016, vol. 31, 65-71
- Novel expandable gastro retentive system by unfolding mechanism of levetiracetam using simple lattice design-Formulation optimization and in vitro evaluation. **SIVANESWARI et al.** Bull Faculty Pharmacy. Cairo University, 2017, vol. 55, 63-72
- **KONG E SINGH**. A Model Stomach System to Investigate Disintegration Kinetics of Solid Foods during Gastric Digestion. *J Food Sci*, 2008, vol. 73 (5), E202-10
- **CASCONE et al.** Mimicking the contractions of a human stomach and their effect on pharmaceuticals. *J Drug Deliv Sci Tech*, 2017, vol. 41, 454-461
- **LEE et al.** Application of an Artificial Stomach-Duodenum Reduced Gastric pH Dog Model for Formulation Principle Assessment and Mechanistic Performance Understanding. *J Pharm Sci*, 2017, vol. 106 (8), 1987-1997
- **CAVIGLIOLI et al.** An innovative matrix controlling drug delivery produced by thermal treatment of DC tablets containing Polycarbophil and ethylcellulose. *Int J Pharm*, 2013, vol. 458 (1), 74-82
- *CHEMICAL ABSTRACTS*, 9004-67-5
- *CHEMICAL ABSTRACTS*, 9004-57-3
- *CHEMICAL ABSTRACTS*, 9004-38-0
- *CHEMICAL ABSTRACTS*, 9050-31-1
- *CHEMICAL ABSTRACTS*, 9002-89-5
- Pharmaceutical Excipients. Pharmaceutical Press
- *CHEMICAL ABSTRACTS*, 25322-68-3
- *CHEMICAL ABSTRACTS*, 9050-04-8
- *CHEMICAL ABSTRACTS*, 9004-32-4

- *CHEMICAL ABSTRACTS*, 9005-80-5
- *CHEMICAL ABSTRACTS*, 9012-76-4
- *CHEMICAL ABSTRACTS*, 9000-30-0
- *CHEMICAL ABSTRACTS*, 9000-65-1
- *CHEMICAL ABSTRACTS*, 9003-01-04
- *CHEMICAL ABSTRACTS*, 96827-24-6
- *CHEMICAL ABSTRACTS*, 9005-32-7
- *CHEMICAL ABSTRACTS*, 9003-11-6
- Pharmaceutical Dosage Forms:Tablets. 1989, vol. 1,2,3
- Pharmaceutical Dosage Forms: Tablets. 1989, vol. 1
- Remington: The Science and Practice of Pharmacy. Pharmaceutical Press, 2011, 903
- **KEENER et al.** Thermal decomposition of sodium bicarbonate. *Chem. Eng. Commun.*, 1985, vol. 33, 93-105